# EUROPEAN PATENT SPECIFICATION

(11) **EP 2 940 032 B1**
(45) Date of publication and mention of the grant of the patent: **25.04.2018**
(21) Application number: 13867712.5
(22) Date of filing: 18.12.2013
(51) Int. Cl.: C07K 1/14, C12P 21/02, C07K 14/435

(54) **EXTRACTION METHOD FOR HYDROPHILIC RECOMBINANT PROTEIN**
EXTRAKTIONSVERFAHREN FÜR HYDROPHILE REKOMBINANTE PROTEINE
PROCÉDÉ D'EXTRACTION DE PROTÉINE RECOMBINANTE HYDROPHILE

(30) Priority: 27.12.2012 JP 2012285392; 26.04.2013 JP 2013093934
(43) Date of publication of application: 04.11.2015
(73) Proprietor: Spiber Inc., Yamagata 997-0052 (JP)
(72) Inventor: OSAWA, Toshiaki, Tsuruoka-shi, Yamagata 997-0052 (JP); MORITA, Keisuke, Tsuruoka-shi, Yamagata 997-0052 (JP)
(74) Representative: Jones, Elizabeth Louise
(86) International application number: PCT/JP2013/083971
(87) International publication number: WO 2014/103846

(56) References cited:
- WO-A1-02/096931
- WO-A2-2011/133886
- JP-A- 2011 504 374
- US-A1- 2002 081 286
- US-A1- 2005 158 821
- M. Nic ET AL: "dipolar aprotic solvent" In: "IUPAC Compendium of Chemical Terminology", 24 February 2014 (2014-02-24), IUPAC, Research Triagle Park, NC, XP055299347, ISBN: 978-0-9678550-9-7 DOI: 10.1351/goldbook.D01751, * the whole document *
- CHANG N ET AL: "Protein separation and purification in neat dimethyl sulfoxide", BIOCHEMICAL AND BIOPHYSICAL RESEARCH COMMUNICATIONS, ACADEMIC PRESS INC. ORLANDO, FL, US, vol. 176, no. 3, 15 May 1991 (1991-05-15), pages 1462-1468, XP024835967, ISSN: 0006-291X, DOI: 10.1016/0006-291X(91)90451-C [retrieved on 1991-05-15]

### SEQUENCE LISTING

<110> Spiber Inc.
<120> THE EXTRACTION METHOD OF HYDROPHILIC PROTEINS
<130> H4189
<150> JP 2012-285392
   <151> 2012-12-27
<150> JP2013-93934
   <151> 2013-04-26
<160> 17
<170> PatentIn version 3.3
<210> 1
   <211> 136
   <212> PRT
   <213> Artificial
<220>
   <223> ADF3Kai-small-M
<400> 1
   Met His His His His His His His His His His Ser Ser Gly Ser Ser
   1               5                   10                  15
   Leu Glu Val Leu Phe Gln Gly Pro Ala Arg Ala Gly Ser Gly Gln Gln
               20                  25                  30
   Gly Pro Gly Gln Gln Gly Pro Gly Gln Gln Gly Pro Gly Gln Gln Gly
           35                  40                  45
   Pro Tyr Gly Pro Gly Ala Ser Ala Ala Ala Ala Ala Ala Gly Gly Tyr
       50                  55                  60
   Gly Pro Gly Ser Gly Gln Gln Gly Pro Ser Gln Gln Gly Pro Gly Gln
   65                  70                  75                  80
   Gln Gly Pro Gly Gly Gln Gly Pro Tyr Gly Pro Gly Ala Ser Ala Ala
                   85                  90                  95
   Ala Ala Ala Ala Gly Gly Tyr Gly Pro Gly Ser Gly Gln Gln Gly Pro
               100                 105                 110
   Gly Gln Gln Gly Pro Gly Gln Gln Gly Pro Gly Gly Gln Gly Pro Tyr
           115                 120                 125
   Gly Pro Gly Ala Ala Ser Ala Ala
       130                 135
<210> 2
   <211> 542
   <212> PRT
   <213> Artificial
<220>
   <223> ADF3Kai-NN
<400> 2
   Met His His His His His His His His His His Ser Ser Gly Ser Ser
   1               5                   10                  15
   Leu Glu Val Leu Phe Gln Gly Pro Ala Arg Ala Gly Ser Gly Gln Gln
               20                  25                  30
   Gly Pro Gly Gln Gln Gly Pro Gly Gln Gln Gly Pro Gly Gln Gln Gly
           35                  40                  45
   Pro Tyr Gly Pro Gly Ala Ser Ala Ala Ala Ala Ala Ala Gly Gly Tyr
       50                  55                  60
   Gly Pro Gly Ser Gly Gln Gln Gly Pro Ser Gln Gln Gly Pro Gly Gln
   65                  70                  75                  80
   Gln Gly Pro Gly Gly Gln Gly Pro Tyr Gly Pro Gly Ala Ser Ala Ala
                   85                  90                  95
   Ala Ala Ala Ala Gly Gly Tyr Gly Pro Gly Ser Gly Gln Gln Gly Pro
               100                 105                 110
   Gly Gly Gln Gly Pro Tyr Gly Pro Gly Ser Ser Ala Ala Ala Ala Ala
           115                 120                 125
   Ala Gly Gly Asn Gly Pro Gly Ser Gly Gln Gln Gly Ala Gly Gln Gln
       130                 135                 140
   Gly Pro Gly Gln Gln Gly Pro Gly Ala Ser Ala Ala Ala Ala Ala Ala
   145                 150                 155                 160
   Gly Gly Tyr Gly Pro Gly Ser Gly Gln Gln Gly Pro Gly Gln Gln Gly
                   165                 170                 175
   Pro Gly Gly Gln Gly Pro Tyr Gly Pro Gly Ala Ser Ala Ala Ala Ala
               180                 185                 190
   Ala Ala Gly Gly Tyr Gly Pro Gly Ser Gly Gln Gly Pro Gly Gln Gln
           195                 200                 205
   Gly Pro Gly Gly Gln Gly Pro Tyr Gly Pro Gly Ala Ser Ala Ala Ala
       210                 215                 220
   Ala Ala Ala Gly Gly Tyr Gly Pro Gly Ser Gly Gln Gln Gly Pro Gly
   225                 230                 235                 240
   Gln Gln Gly Pro Gly Gln Gln Gly Pro Gly Gly Gln Gly Pro Tyr Gly
                   245                 250                 255
   Pro Gly Ala Ser Ala Ala Ala Ala Ala Ala Gly Gly Tyr Gly Pro Gly
               260                 265                 270
   Tyr Gly Gln Gln Gly Pro Gly Gln Gln Gly Pro Gly Gly Gln Gly Pro
           275                 280                 285
   Tyr Gly Pro Gly Ala Ser Ala Ala Ser Ala Ala Ser Gly Gly Tyr Gly
       290                 295                 300
   Pro Gly Ser Gly Gln Gln Gly Pro Gly Gln Gln Gly Pro Gly Gly Gln
   305                 310                 315                 320
   Gly Pro Tyr Gly Pro Gly Ala Ser Ala Ala Ala Ala Ala Ala Gly Gly
                   325                 330                 335
   Tyr Gly Pro Gly Ser Gly Gln Gln Gly Pro Gly Gln Gln Gly Pro Gly
               340                 345                 350
   Gln Gln Gly Pro Gly Gln Gln Gly Pro Gly Gly Gln Gly Pro Tyr Gly
           355                 360                 365
   Pro Gly Ala Ser Ala Ala Ala Ala Ala Ala Gly Gly Tyr Gly Pro Gly
       370                 375                 380
   Ser Gly Gln Gln Gly Pro Gly Gln Gln Gly Pro Gly Gln Gln Gly Pro
   385                 390                 395                 400
   Gly Gln Gln Gly Pro Gly Gln Gln Gly Pro Gly Gln Gln Gly Pro Gly
                   405                 410                 415
   Gln Gln Gly Pro Gly Gln Gln Gly Pro Gly Gln Gln Gly Pro Gly Gly
               420                 425                 430
   Gln Gly Ala Tyr Gly Pro Gly Ala Ser Ala Ala Ala Gly Ala Ala Gly
           435                 440                 445
   Gly Tyr Gly Pro Gly Ser Gly Gln Gln Gly Pro Gly Gln Gln Gly Pro
       450                 455                 460
   Gly Gln Gln Gly Pro Gly Gln Gln Gly Pro Gly Gln Gln Gly Pro Gly
   465 470 475 480
   Gln Gln Gly Pro Gly Gln Gln Gly Pro Gly Gln Gln Gly Pro Tyr Gly
   485 490 495
   Pro Gly Ala Ser Ala Ala Ala Ala Ala Ala Gly Gly Tyr Gly Pro Gly
   500 505 510
   Ser Gly Gln Gln Gly Pro Gly Gln Gln Gly Pro Gly Gln Gln Gly Pro
   515 520 525
   Gly Gly Gln Gly Pro Tyr Gly Pro Gly Ala Ala Ser Ala Ala
   530 535 540
<210> 3
   <211> 24
   <212> PRT
   <213> Artificial
<220>
   <223> His tag
<400> 3
   Met His His His His His His His His His His Ser Ser Gly Ser Ser
   1 5 10 15
   Leu Glu Val Leu Phe Gln Gly Pro
   20
<210> 4
   <211> 408
   <212> DNA
   <213> Artificial
<220>
   <223> ADF3Kai-small-M
<400> 4
   atgcaccacc accatcacca tcatcatcac cactcatcag gttcaagcct tgaagtatta 60
   tttcagggac ccgcacgcgc aggctcaggg cagcagggtc cgggtcagca ggggcctgga 120
   caacaaggac cagggcaaca ggggccctac gggccaggag cttcagcagc agctgcggct 180
   gccggtggtt atggacccgg cagtggccag caaggcccca gccaacaagg tccaggacag 240
   cagggcccag gtggtcaagg accttacgga cctggtgcga gcgcggcagc ggccgccgcg 300
   ggaggttacg gccccggtag cggtcaacag ggtcctggtc agcaaggtcc cggccaacag 360
   ggaccgggcg gccaagggcc ttatgggccg ggcgcagctt ccgcagcg 408
<210> 5
   <211> 1626
   <212> DNA
   <213> Artificial
<220>
   <223> ADF3Kai-NN
<400> 5
   atgcatcacc atcatcatca tcaccaccac cattcctcgg gctcatcctt ggaagtgtta     60
   tttcaaggac cagcacgagc cggttcggga caacaagggc ctggccagca gggcccaggt    120
   caacaagggc caggacagca gggtccttat gggcccggcg caagcgcagc agctgcggcc    180
   gctggtggct atggtcctgg ctccggtcaa cagggccctt cgcaacaagg tcccgggcag    240
   caaggtcctg gtggccaggg tccctacggg ccgggggcga gtgcggcagc agccgctgca    300
   ggcggttatg gtccaggaag cggacagcaa ggtccgggag gtcaaggtcc gtatggccca    360
   ggctctagcg cggctgccgc tgccgcgggt ggcaacggac cagggagcgg acaacagggc    420
   gcgggacaac agggtccagg acagcaaggc ccaggggcgt cggcggctgc agcggcggcc    480
   ggaggctatg gacccggctc aggacaacag ggaccgggtc aacaaggacc cggtggccaa    540
   ggcccctatg gcccgggcgc cagcgcggcc gcagccgccg cgggcgggta cggccccggt    600
   agcggccagg gaccaggtca gcaggggcca ggaggtcagg gcccatacgg tccgggcgca    660
   tccgcggcgg cggcagcggc aggtggctac ggtcccggaa gcggccaaca ggggccaggg    720
   caacaaggac caggacaaca aggtcctggg ggccaaggac cgtatggacc aggagcatca    780
   gctgcagccg cggcagctgg cggttacggt ccaggctacg gccagcaggg tccgggtcag    840
   cagggaccgg gaggccaggg gccttatggc cctggcgctt ccgcagccag tgccgcttct    900
   ggaggatacg ggccgggaag cggtcagcaa ggccctggcc aacaaggacc tggaggccaa    960
   gggccctacg gcccaggagc ctcggcagcc gcagctgccg caggtgggta tgggccaggt   1020
   agcgggcaac aagggccggg tcagcaagga ccggggcaac agggacctgg gcagcaagga   1080
   cccgggggtc aaggcccgta cggacctggt gcgtctgcag ctgctgctgc ggctggtgga   1140
   tatggtccgg gatcggggca gcagggtccc ggtcagcagg gccctggtca gcaagggcca   1200
   ggccaacagg gacccggaca acaaggcccg ggtcaacagg gtcctggaca gcaggggccg   1260
   ggccaacaag gccctgggca acagggtccg gggggacagg gggcctatgg gcctggcgca   1320
   tctgccgccg ctggcgcagc cggtgggtac gggcctgggt caggtcaaca ggggcctggt   1380
   caacaaggcc ccgggcaaca gggccccggc cagcaaggtc cagggcagca gggcccggga   1440
   cagcaagggc ctggacaaca ggggcccgga cagcagggac cttacgggcc cggtgcgagc   1500
   gcagcggccg ccgccgcagg gggatatggc cccggatcgg gccagcaggg accaggccag   1560
   caaggacctg gccaacaggg cccggggggt caggggccgt atggtcccgg cgctgcaagt    1620
   gctgca                                                              1626
<210> 6
   <211> 318
   <212> PRT
   <213> Artificial
<220>
   <223> ADF4Kai-W
<400> 6
   Met His His His His His His His His His His Ser Ser Gly Ser Ser
   1               5                   10                  15
   Leu Glu Val Leu Phe Gln Gly Pro Ala Gly Ser Ser Ala Ala Ala Ala
               20                  25                  30
   Ala Ala Ala Ser Gly Ser Gly Gly Tyr Gly Pro Glu Asn Gln Gly Pro
           35                  40                  45
   Ser Gly Pro Val Ala Tyr Gly Pro Gly Gly Pro Val Ser Ser Ala Ala
       50                  55                  60
   Ala Ala Ala Ala Ala Gly Ser Gly Pro Gly Gly Tyr Gly Pro Glu Asn
   65                  70                  75                  80
   Gln Gly Pro Ser Gly Pro Gly Gly Tyr Gly Pro Gly Gly Ser Gly Ser
                   85                  90                  95
   Ser Ala Ala Ala Ala Ala Ala Ala Ala Ser Gly Pro Gly Gly Tyr Gly
               100                 105                 110
   Pro Gly Ser Gln Gly Pro Ser Gly Pro Gly Gly Ser Gly Gly Tyr Gly
           115                 120                 125
   Pro Gly Ser Gln Gly Ala Ser Gly Pro Gly Gly Pro Gly Ala Ser Ala
       130                 135                 140
   Ala Ala Ala Ala Ala Ala Ala Ala Ala Ser Gly Pro Gly Gly Tyr Gly
   145                 150                 155                 160
   Pro Gly Ser Gln Gly Pro Ser Gly Pro Gly Ala Tyr Gly Pro Gly Gly
                   165                 170                 175
   Pro Gly Ser Ser Ala Ala Ala Ala Ala Ala Ala Ala Ser Gly Pro Gly
               180                 185                 190
   Gly Tyr Gly Pro Gly Ser Gln Gly Pro Ser Gly Pro Gly Val Tyr Gly
           195                 200                 205
   Pro Gly Gly Pro Gly Ser Ser Ala Ala Ala Ala Ala Ala Ala Gly Ser
       210                 215                 220
   Gly Pro Gly Gly Tyr Gly Pro Glu Asn Gln Gly Pro Ser Gly Pro Gly
   225                 230                 235                 240
   Gly Tyr Gly Pro Gly Gly Ser Gly Ser Ser Ala Ala Ala Ala Ala Ala
                   245                 250                 255
   Ala Ala Ser Gly Pro Gly Gly Tyr Gly Pro Gly Ser Gln Gly Pro Ser
               260                 265                 270
   Gly Pro Gly Gly Ser Gly Gly Tyr Gly Pro Gly Ser Gln Gly Gly Ser
           275                 280                 285
   Gly Pro Gly Ala Ser Ala Ala Ala Ala Ala Ala Ala Ala Ser Gly Pro
       290                 295                 300
   Gly Gly Tyr Gly Pro Gly Ser Gln Gly Pro Ser Gly Pro Gly
   305                 310                 315
<210> 7
   <211> 954
   <212> DNA
   <213> Artificial
<220>
   <223> ADF4Kai-W
<400> 7
   atgcaccatc atcatcacca tcaccaccat catagcagtg gatcttccct cgaagtactc      60
   tttcaaggtc cagcaggtag tagcgctgcc gcagctgcag cagcttcagg ttccggtggc     120
   tacgggccag aaaatcaggg tccgagcggt ccggttgcgt atggcccagg tggccctgtg     180
   agcagcgcag cagcagccgc agcagctggt agcggacccg ggggctacgg ccctgagaat     240
   caaggaccga gtggcccggg gggttatggt cctggaggta gtgggagcag cgcggcggct     300
   gcagcagcgg cggcgagtgg gccaggtgga tatgggccag gctcccaggg gcctagtggc     360
   cctggtggtt caggagggta tggaccagga tcacagggcg catccggacc tggcggcccg     420
   ggtgcaagtg ctgcggcggc cgcagctgct gctgccgcca gtggaccagg cgggtatggc     480
   cctggatccc aaggtccatc gggaccagga gcatacgggc ctgggggacc aggctccagt     540
   gcggccgcgg cggcggcggc agcctccggt ccaggaggtt atggaccggg aagtcaaggc     600
   ccttctggac ctggagtgta cggtccaggc ggaccgggtt cgagtgctgc agcggcagcg     660
   gccgcaggtt cgggccccgg aggctacgga ccagagaatc aggggccgtc tggcccggga     720
   ggctatggtc cgggcggctc cggttccagt gcagcggctg ccgcggctgc ggcgtcaggc    780
   cctggcggtt atggccccgg tagccaaggt cccagtggtc cgggaggaag cggcggttac    840
   ggtccgggca gccaaggtgg ctcaggtcct ggtgccagcg cggctgccgc agcggcagct    900
   gcgtcaggtc caggtggtta tgggccgggt agccaggggc catctggtcc tggg          954
<210> 8
   <211> 467
   <212> PRT
   <213> Artificial
<220>
   <223> Flag92-short2-UU
<400> 8
   Met His His His His His His His His His His Ser Ser Gly Ser Ser
   1               5                   10                  15
   Leu Glu Val Leu Phe Gln Gly Pro Gly Ala Gly Gly Ser Gly Pro Gly
               20                  25                  30
   Gly Ala Gly Pro Gly Gly Val Gly Pro Gly Gly Ser Gly Pro Gly Gly
           35                  40                  45
   Val Gly Pro Gly Gly Ser Gly Pro Gly Gly Val Gly Pro Gly Gly Ser
       50                  55                  60
   Gly Pro Gly Gly Val Gly Pro Gly Gly Ala Gly Gly Pro Tyr Gly Pro
   65                  70                  75                  80
   Gly Gly Ser Gly Pro Gly Gly Ala Gly Gly Ala Gly Gly Pro Gly Gly
                   85                  90                  95
   Ala Tyr Gly Pro Gly Gly Ser Tyr Gly Pro Gly Gly Ser Gly Gly Pro
               100                 105                 110
   Gly Gly Ala Gly Gly Pro Tyr Gly Pro Gly Gly Glu Gly Pro Gly Gly
           115                 120                 125
   Ala Gly Gly Pro Tyr Gly Pro Gly Gly Ala Gly Gly Pro Tyr Gly Pro
       130                 135                 140
   Gly Gly Ala Gly Gly Pro Tyr Gly Pro Gly Gly Glu Gly Gly Pro Tyr
   145                 150                 155                 160
   Gly Pro Gly Gly Ser Tyr Gly Pro Gly Gly Ala Gly Gly Pro Tyr Gly
                   165                 170                 175
   Pro Gly Gly Pro Tyr Gly Pro Gly Gly Glu Gly Pro Gly Gly Ala Gly
               180                 185                 190
   Gly Pro Tyr Gly Pro Gly Gly Val Gly Pro Gly Gly Gly Gly Pro Gly
           195                 200                 205
   Gly Tyr Gly Pro Gly Gly Ala Gly Pro Gly Gly Tyr Gly Pro Gly Gly
       210                 215                 220
   Ser Gly Pro Gly Gly Tyr Gly Pro Gly Gly Ser Gly Pro Gly Gly Tyr
   225                 230                 235                     240
   Gly Pro Gly Gly Ser Gly Pro Gly Gly Tyr Gly Pro Gly Gly Ser Gly
                   245                 250                 255
   Pro Gly Gly Tyr Gly Pro Gly Gly Ser Gly Pro Gly Gly Ser Gly Pro
               260                 265                 270
   Gly Gly Tyr Gly Pro Gly Gly Ser Gly Pro Gly Gly Ser Gly Pro Gly
           275                 280                 285
   Gly Tyr Gly Pro Gly Gly Ser Gly Pro Gly Gly Tyr Gly Pro Gly Gly
       290                 295                 300
   Ser Gly Pro Gly Gly Ser Gly Pro Gly Gly Tyr Gly Pro Gly Gly Ser
   305                 310                 315                     320
   Gly Pro Gly Gly Ser Gly Pro Gly Gly Tyr Gly Pro Gly Gly Ser Gly
                   325                 330                 335
   Pro Gly Gly Phe Gly Pro Gly Gly Phe Gly Pro Gly Gly Ser Gly Pro
               340                 345                 350
   Gly Gly Tyr Gly Pro Gly Gly Ser Gly Pro Gly Gly Ala Gly Pro Gly
           355                 360                 365
   Gly Val Gly Pro Gly Gly Phe Gly Pro Gly Gly Ala Gly Pro Gly Gly
       370                 375                 380
   Ala Gly Pro Gly Gly Ala Gly Pro Gly Gly Ala Gly Pro Gly Gly Ala
   385                 390                 395                     400
   Gly Pro Gly Gly Ala Gly Pro Gly Gly Ala Gly Pro Gly Gly Ala Gly
                   405                 410                 415
   Pro Gly Gly Ala Gly Gly Ala Gly Gly Ala Gly Gly Ala Gly Gly Ser
               420                 425                 430
   Gly Gly Ala Gly Gly Ser Gly Gly Thr Thr Ile Ile Glu Asp Leu Asp
           435                 440                 445
   Ile Thr Ile Asp Gly Ala Asp Gly Pro Ile Thr Ile Ser Glu Glu Leu
       450                 455                 460
   Thr Ile Ser
   465
<210> 9
   <211> 1401
   <212> DNA
   <213> Artificial
<220>
   <223> Flag92-short2-UU
<400> 9
   atgcatcacc atcatcatca tcaccaccac cattcctcgg gctcatcctt ggaagtgtta      60
   tttcaaggac caggagctgg agggtccggt ccgggcggcg ctggtccggg aggagtgggt     120
   ccgggtggtt ctggacccgg tggagtaggt ccgggcggtt ccggtccggg aggcgtcgga     180
   cctggtggat caggacctgg aggtgtaggt cctggtggcg cgggtggccc ttacggccct     240
   gggggctctg gcccaggagg cgctggtggg gcaggagggc ctggtggggc gtatggtccc     300
   gggggttcat acggcccagg gggttcagga ggtccaggag gtgcaggggg accatacgga     360
   ccgggcggtg agggaccagg gggcgctggc ggaccctatg gacctggcgg agctggaggt     420
   ccctacgggc caggcggggc tggaggaccg tatggaccgg gtggagaagg tggtccgtac     480
   ggcccgggcg ggtcttacgg accaggtggc gcaggaggtc cttatgggcc gggtggacca     540
   tacggtccag gcggagaggg tccaggtggg gcaggtgggc cttatggccc gggaggagtt     600
   ggtccaggcg gcggtggtcc gggcgggtat ggcccgggtg gcgcaggtcc aggcggatat     660
   ggaccaggtg gctctggacc cggagggtac gggccgggtg gttcagggcc tggaggttat     720
   ggtccgggag gtagtggacc aggaggttat gggcctgggg gttcagggcc aggtggttat     780
   ggtccaggag gatcaggtcc aggtggaagt ggtccagggg gttatggccc tggtggtagc     840
   ggcccaggtg gcagtggacc gggaggatat ggcccaggag gttcaggtcc cggtggatac     900
   ggaccgggtg gatctggacc aggcggttct ggtccgggag gttacggtcc tgggggtagt     960
   ggtcccggag gtagtggacc aggtggatat ggaccaggag ggtctggacc cggaggtttt    1020
   ggtccaggcg gatttggtcc tggtggttct gggcctggtg ggtatggtcc tggcggatct    1080
   ggtccaggtg gggcaggacc tggtggagtt ggacctgggg gttttggacc cggtggtgcg    1140
   ggaccagggg gtgcaggtcc tggtggcgct ggtcctggag gcgctgggcc aggcggtgct    1200
   ggacctggag gagcaggacc aggtggtgct ggccccggtg gcgcaggccc tggaggtgct    1260
   ggtggagctg gtggtgcagg tggagcaggt ggttcaggag gagctggtgg ttcaggtgga    1320
   actactataa tcgaagacct tgatattacg attgatggag cagatggtcc catcactatt    1380
   tcggaagaat tgactatttc a                                              1401
<210> 10
   <211> 310
   <212> PRT
   <213> Artificial
<220>
   <223> Resilin-Kai
<400> 10
   Met His His His His His His Pro Glu Pro Pro Val Asn Ser Tyr Leu
   1               5                   10                  15
   Pro Pro Ser Asp Ser Tyr Gly Ala Pro Gly Gln Ser Gly Pro Gly Gly
               20                  25                  30
   Arg Pro Ser Asp Ser Tyr Gly Ala Pro Gly Gly Gly Asn Gly Gly Arg
           35                  40                  45
   Pro Ser Asp Ser Tyr Gly Ala Pro Gly Gln Gly Gln Gly Gln Gly Gln
       50                  55                  60
   Gly Gln Gly Gly Tyr Ala Gly Lys Pro Ser Asp Ser Tyr Gly Ala Pro
   65                  70                  75                  80
   Gly Gly Gly Asp Gly Asn Gly Gly Arg Pro Ser Ser Ser Tyr Gly Ala
                   85                  90                  95
   Pro Gly Gly Gly Asn Gly Gly Arg Pro Ser Asp Thr Tyr Gly Ala Pro
               100                 105                 110
   Gly Gly Gly Asn Gly Gly Arg Pro Ser Asp Thr Tyr Gly Ala Pro Gly
           115                 120                 125
   Gly Gly Gly Asn Gly Asn Gly Gly Arg Pro Ser Ser Ser Tyr Gly Ala
       130                 135                 140
   Pro Gly Gln Gly Gln Gly Asn Gly Asn Gly Gly Arg Pro Ser Ser Ser
   145                 150                 155                 160
   Tyr Gly Ala Pro Gly Gly Gly Asn Gly Gly Arg Pro Ser Asp Thr Tyr
                   165                 170                 175
   Gly Ala Pro Gly Gly Gly Asn Gly Gly Arg Pro Ser Asp Thr Tyr Gly
                   180                 185                 190
   Ala Pro Gly Gly Gly Asn Asn Gly Gly Arg Pro Ser Ser Ser Tyr Gly
           195                 200                 205
   Ala Pro Gly Gly Gly Asn Gly Gly Arg Pro Ser Asp Thr Tyr Gly Ala
       210                 215                 220
   Pro Gly Gly Gly Asn Gly Asn Gly Ser Gly Gly Arg Pro Ser Ser Ser
   225                 230                 235                 240
   Tyr Gly Ala Pro Gly Gln Gly Gln Gly Gly Phe Gly Gly Arg Pro Ser
                   245                 250                 255
   Asp Ser Tyr Gly Ala Pro Gly Gln Asn Gln Lys Pro Ser Asp Ser Tyr
               260                 265                 270
   Gly Ala Pro Gly Ser Gly Asn Gly Asn Gly Gly Arg Pro Ser Ser Ser
           275                 280                 285
   Tyr Gly Ala Pro Gly Ser Gly Pro Gly Gly Arg Pro Ser Asp Ser Tyr
       290                 295                 300
   Gly Pro Pro Ala Ser Gly
   305                 310
<210> 11
   <211> 930
   <212> DNA
   <213> Artificial
<220>
   <223> Resilin-Kai
<400> 11
   atgcatcatc accaccatca tccggaacct cctgtcaaca gctatttgcc ccctagtgat    60
   tcttacggag caccaggtca gtctggacca ggcgggagac cttctgacag ttacggcgct   120
   cctggtggcg gcaatggtgg gaggccatct gatagctacg gagccccagg ccaagggcag   180
   ggtcagggcc aaggtcaagg tggctacgct ggtaaaccgt cggactctta tggtgcacct   240
   ggcggggggg acggaaacgg tggcagaccc agcagttcct atggggcacc cggtggaggc   300
   aatgggggcc gacctagcga cacttacggg gcccccggcg gcgggaacgg tgggcgtccc   360
   agcgatacct acggtgcccc gggtggggga gggaacggca acgggggaag gcctagttct   420
   agttatggcg cgccgggcca gggacaaggg aatggtaacg gagggcgccc atccagctcg   480
   tatggcgctc ccgggggtgg taatgggggt cgcccttcag atacatacgg ggctccaggg   540
   gggggtaacg gcggacggcc ctcggacacc tatggagctc cgggcggagg aaataatgga   600
   ggccgcccaa gttcatctta tggagcgccc ggtggtggaa acggcggtag accgtcagac   660
   acgtatgggg cgcctggagg cggtaatggc aacggttcgg gaggtaggcc gagctcaagc   720
   tatggtgcgc cagggcaagg acagggcggt tttggcggaa gaccatccga tagttacggt   780
   gctcctgggc agaaccaaaa accttcggat tcatacggcg cccctggtag tgggaatgga   840
   aatggcggtc gtccatcgtc ctcctacggc gcaccgggaa gcggtcccgg aggtcgaccg   900
   agtgactcgt acggtccacc tgcaagtggt                                    930
<210> 12
   <211> 7
   <212> PRT
   <213> Artificial
<220>
   <223> His Tag6
<400> 12
   Met His His His His His His
   1               5
<210> 13
   <211> 310
   <212> PRT
   <213> Artificial Sequence
<220>
   <223> Drosophila-Resilin-Kai
<400> 13
   Met His His His His His His Pro Glu Pro Pro Val Asn Ser Tyr Leu
   1               5                   10                  15
   Pro Pro Ser Asp Ser Tyr Gly Ala Pro Gly Gln Ser Gly Pro Gly Gly
               20                  25                  30
   Arg Pro Ser Asp Ser Tyr Gly Ala Pro Gly Gly Gly Asn Gly Gly Arg
           35                  40                  45
   Pro Ser Asp Ser Tyr Gly Ala Pro Gly Gln Gly Gln Gly Gln Gly Gln
       50                  55                  60
   Gly Gln Gly Gly Tyr Ala Gly Lys Pro Ser Asp Ser Tyr Gly Ala Pro
   65                  70                  75                  80
   Gly Gly Gly Asp Gly Asn Gly Gly Arg Pro Ser Ser Ser Tyr Gly Ala
                   85                  90                  95
   Pro Gly Gly Gly Asn Gly Gly Arg Pro Ser Asp Thr Tyr Gly Ala Pro
                   100                 105                 110
   Gly Gly Gly Asn Gly Gly Arg Pro Ser Asp Thr Tyr Gly Ala Pro Gly
           115                 120                 125
   Gly Gly Gly Asn Gly Asn Gly Gly Arg Pro Ser Ser Ser Tyr Gly Ala
       130                 135                 140
   Pro Gly Gln Gly Gln Gly Asn Gly Asn Gly Gly Arg Pro Ser Ser Ser
   145                 150                 155                 160
   Tyr Gly Ala Pro Gly Gly Gly Asn Gly Gly Arg Pro Ser Asp Thr Tyr
                   165                 170                 175
   Gly Ala Pro Gly Gly Gly Asn Gly Gly Arg Pro Ser Asp Thr Tyr Gly
               180                 185                 190
   Ala Pro Gly Gly Gly Asn Asn Gly Gly Arg Pro Ser Ser Ser Tyr Gly
           195                 200                 205
   Ala Pro Gly Gly Gly Asn Gly Gly Arg Pro Ser Asp Thr Tyr Gly Ala
       210                 215                 220
   Pro Gly Gly Gly Asn Gly Asn Gly Ser Gly Gly Arg Pro Ser Ser Ser
   225                 230                 235                 240
   Tyr Gly Ala Pro Gly Gln Gly Gln Gly Gly Phe Gly Gly Arg Pro Ser
                   245                 250                 255
   Asp Ser Tyr Gly Ala Pro Gly Gln Asn Gln Lys Pro Ser Asp Ser Tyr
               260                 265                 270
   Gly Ala Pro Gly Ser Gly Asn Gly Asn Gly Gly Arg Pro Ser Ser Ser
           275                 280                 285
   Tyr Gly Ala Pro Gly Ser Gly Pro Gly Gly Arg Pro Ser Asp Ser Tyr
       290                 295                 300
   Gly Pro Pro Ala Ser Gly
   305                 310
<210> 14
   <211> 930
   <212> DNA
   <213> Artificial Sequence
<220>
   <223> Drosophila-Resilin-Kai
<400> 14
   atgcatcatc accaccatca tccggaacct cctgtcaaca gctatttgcc ccctagtgat      60
   tcttacggag caccaggtca gtctggacca ggcgggagac cttctgacag ttacggcgct     120
   cctggtggcg gcaatggtgg gaggccatct gatagctacg gagccccagg ccaagggcag     180
   ggtcagggcc aaggtcaagg tggctacgct ggtaaaccgt cggactctta tggtgcacct     240
   ggcggggggg acggaaacgg tggcagaccc agcagttcct atggggcacc cggtggaggc     300
   aatgggggcc gacctagcga cacttacggg gcccccggcg gcgggaacgg tgggcgtccc     360
   agcgatacct acggtgcccc gggtggggga gggaacggca acgggggaag gcctagttct     420
   agttatggcg cgccgggcca gggacaaggg aatggtaacg gagggcgccc atccagctcg     480
   tatggcgctc ccgggggtgg taatgggggt cgcccttcag atacatacgg ggctccaggg     540
   gggggtaacg gcggacggcc ctcggacacc tatggagctc cgggcggagg aaataatgga     600
   ggccgcccaa gttcatctta tggagcgccc ggtggtggaa acggcggtag accgtcagac     660
   acgtatgggg cgcctggagg cggtaatggc aacggttcgg gaggtaggcc gagctcaagc     720
   tatggtgcgc cagggcaagg acagggcggt tttggcggaa gaccatccga tagttacggt     780
   gctcctgggc agaaccaaaa accttcggat tcatacggcg cccctggtag tgggaatgga     840
   aatggcggtc gtccatcgtc ctcctacggc gcaccgggaa gcggtcccgg aggtcgaccg     900
   agtgactcgt acggtccacc tgcaagtggt                                      930
<210> 15
   <211> 12
   <212> **PRT**
   <213> Artificial Sequence
<220>
   <223> HisTag6-II
<400> 15
   Met His His His His His His Ser Ser Gly Ser Ser
   1               5                   10
<210> 16
   <211> 252
   <212> PRT
   <213> Artificial Sequence
<220>
   <223> Collagen-type4-Kai
<400> 16
   Met His His His His His His Ser Ser Gly Ser Ser Lys Asp Gly Val
   1               5                   10                  15
   Pro Gly Phe Pro Gly Ser Glu Gly Val Lys Gly Asn Arg Gly Phe Pro
               20                  25                  30
   Gly Leu Met Gly Glu Asp Gly Ile Lys Gly Gln Lys Gly Asp Ile Gly
           35                  40                  45
   Pro Pro Gly Phe Arg Gly Pro Thr Glu Tyr Tyr Asp Thr Tyr Gln Glu
       50                  55                  60
   Lys Gly Asp Glu Gly Thr Pro Gly Pro Pro Gly Pro Arg Gly Ala Arg
   65                  70                  75                  80
   Gly Pro Gln Gly Pro Ser Gly Pro Pro Gly Val Pro Gly Ser Pro Gly
                   85                  90                  95
   Ser Ser Arg Pro Gly Leu Arg Gly Ala Pro Gly Trp Pro Gly Leu Lys
               100                 105                 110
   Gly Ser Lys Gly Glu Arg Gly Arg Pro Gly Lys Asp Ala Met Gly Thr
           115                 120                 125
   Pro Gly Ser Pro Gly Cys Ala Gly Ser Pro Gly Leu Pro Gly Ser Pro
       130                 135                 140
   Gly Pro Pro Gly Pro Pro Gly Asp Ile Val Phe Arg Lys Gly Pro Pro
   145                 150                 155                 160
   Gly Asp His Gly Leu Pro Gly Tyr Leu Gly Ser Pro Gly Ile Pro Gly
                   165                 170                 175
   Val Asp Gly Pro Lys Gly Glu Pro Gly Leu Leu Cys Thr Gln Cys Pro
               180                 185                 190
   Tyr Ile Pro Gly Pro Pro Gly Leu Pro Gly Leu Pro Gly Leu His Gly
           195                 200                 205
   Val Lys Gly Ile Pro Gly Arg Gln Gly Ala Ala Gly Leu Lys Gly Ser
       210                 215                 220
   Pro Gly Ser Pro Gly Asn Thr Gly Leu Pro Gly Phe Pro Gly Phe Pro
   225                 230                 235                 240
   Gly Ala Gln Gly Asp Pro Gly Leu Lys Gly Glu Lys
                   245                 250
<210> 17
   <211> 756
   <212> DNA
   <213> Artificial Sequence
<220>
   <223> Collagen-type4-Kai
<400> 17
   atgcaccatc atcaccacca tagctctgga agttcaaaag acggagtacc tgggtttcca     60
   ggttcggagg gggttaaagg aaatcgagga ttccctgggc tactgggaga agacggcata    120
   aaaggtcaga aaggcgacat aggtcctccc ggatttagag gccccacaga atactacgac    180
   acgtatcaag aaaagggaga tgagggcact ccggggccgc ccggcccacg aggggctcga    240
   ggtccccaag gaccgtctgg accaccaggc gtgcctggct cccccggcag ttcccgccca    300
   ggattacgcg gagctccagg atggccaggg ttgaaaggta gtaaaggtga gcgtggccgt    360
   cctgggaaag atgcggttgg cacaccgggg tctcctgggt gcgcgggtag tcccggtctt    420
   ccgggttctc cgggccctcc tggacctcca ggggacattg tgttcaggaa gggtccacct    480
   ggcgatcatg gacttcccgg ctatctcgga agcccgggga tacctggggt agatgggcct    540
   aaaggggaac caggccttct ttgtacccag tgtccgtata taccgggtcc gcccggttta    600
   ccaggtttgc cagggctgca tggtgtcaag ggcattcctg gtcgtcaggg cgcagcgggt    660
   ctcaaaggat cacccgggag tccgggcaac accggcttac ccggctttcc gggttttcct    720
   ggcgcgcaag gtgaccctgg attaaaagga gagaag                              756

## Description

### Technical Field

The present invention relates to a method for extracting a hydrophilic recombinant protein, and specifically, relates to a method for extracting a hydrophilic recombinant protein from a host expressing a hydrophilic recombinant protein such as a recombinant spider silk protein having a hydropathy index of 0 or less.

### Background Art

Recently, genetic recombination technologies have made it possible to introduce target foreign genes into host cells such as Escherichia coli to express target proteins. By applying these technologies, various kinds of useful recombinant proteins have been produced. Spider silk fibers are fibers having high strength and toughness, and have attracted attention as novel materials because of such properties. By genetic recombination technologies, spider silk proteins serving as spider silk raw materials are expressed in host cells such as Escherichia coli and collected. Generally, in the production of recombinant proteins using hosts such as Escherichia coli, bacterial cells are disrupted so as to collect target proteins. For example, Patent Document 1 describes a method for collecting recombinant spider silk proteins from host cells.

### Prior Art Document

### Patent Document

Patent Document 1: JP 2004-503204 A

### Disclosure of Invention

### Problem to be Solved by the Invention

In Patent Document 1, organic acids such as formic acid and propionic acid are used to dissolve host cells so as to collect recombinant spider silk proteins. However, there is a problem that, when organic acids such as formic acid and propionic acid are used, proteins that are not resistant to acids decompose easily.

In order to solve the above conventional problem, it is an object of the present invention to provide a method for extracting a hydrophilic recombinant protein that is capable of extracting a hydrophilic recombinant protein from a host expressing a hydrophilic recombinant protein such as a hydrophilic recombinant spider silk protein without using organic acids.

### Means for Solving Problem

The present invention relates to a method for extracting a hydrophilic recombinant protein from a host expressing a hydrophilic recombinant protein. The method includes: a lysate acquisition step in which a dissolving solvent is added to the host expressing a hydrophilic recombinant protein so as to obtain a host cell lysate; and a step in which a diluting solvent is added to the host cell lysate obtained in the lysate acquisition step, an insoluble substance is separated from the obtained dilution, and a supernatant containing the hydrophilic recombinant protein is collected. The hydrophilic recombinant protein has a hydropathy index of 0 or less. The dissolving solvent is an aprotic polar solvent having a dipole moment of 3.0D or more. The diluting solvent is an aqueous solvent.

In the method for extracting a hydrophilic recombinant protein according to the present invention, preferably, the dissolving solvent is an aprotic polar solvent not having a cyclic structure, and has a dipole moment of 3.0 D or more. More preferably, the dissolving solvent is one or more kinds of aprotic polar solvent selected from the group consisting of dimethyl sulfoxide, N,N-dimethylformamide, and N,N-dimethylacetamide. Preferably, the dissolving solvent is a solvent obtained by adding an inorganic salt to the aprotic polar solvent. Preferably, the diluting solvent is one kind of aqueous solvent selected from the group consisting of (1) water, (2) a mixture of water and ethanol, and (3) a water-soluble buffer solution. Preferably, an addition amount (amount which is added) of the dissolving solvent is 0.5 times or more the mass of the host by volume (mL)/mass (g) ratio. Preferably, an addition amount of the diluting solvent is 0.5 times or more an amount of the host cell lysate by volume ratio. Preferably, the hydrophilic recombinant protein is a protein selected from the group consisting of a recombinant spider silk protein, a recombinant resilin, and a recombinant collagen.

### Effect of the Invention

According to the present invention, by using an aprotic polar solvent having a dipole moment of 3.0D or more as a dissolving solvent; using an aqueous solvent as a diluting solvent; dissolving a host expressing a hydrophilic recombinant protein having a hydropathy index of 0 or less using the dissolving solvent; diluting the obtained host cell lysate by adding the diluting solvent; separating an insoluble substance therefrom; and collecting a supernatant, the hydrophilic recombinant protein having a hydropathy index of 0 or less can be extracted easily and efficiently from the host without using organic acids.

Further, according to the present invention, by using an aprotic polar solvent having a dipole moment of 3.0D or more and not having a cyclic structure, hydrophilic recombinant proteins such as a recombinant spider silk protein, a recombinant resilin and a recombinant collagen having a hydropathy index of 0 or less can be obtained with higher purity. Moreover, according to the present invention, by using a solvent obtained by adding an inorganic salt to the aprotic polar solvent as the dissolving solvent, hydrophilic recombinant proteins such as a recombinant spider silk protein, a recombinant resilin and a recombinant collagen having a hydropathy index of 0 or less can be obtained with higher purity.

### Brief Description of Drawings

[FIG. 1] FIG. 1 is a photograph showing the results of SDS-polyacrylamide gel electrophoresis (SDS-PAGE) of a protein obtained in Example 1.
[FIG. 2] FIG. 2 is a photograph showing the results of SDS-polyacrylamide gel electrophoresis of proteins obtained in Examples 2-5.
[FIG. 3] FIG. 3 is a photograph showing the results of SDS-polyacrylamide gel electrophoresis of proteins obtained in Examples 6-8.
[FIG. 4] FIG. 4 is a photograph showing the results of SDS-polyacrylamide gel electrophoresis of proteins obtained in Examples 9-13.
[FIG. 5] FIG. 5 is a photograph showing the results of SDS-polyacrylamide gel electrophoresis of a protein obtained in Example 16.
[FIG. 6] FIG. 6 is a photograph showing the results of SDS-polyacrylamide gel electrophoresis of proteins obtained in Examples 17-21.
[FIG. 7] FIG. 7 is a photograph showing the results of SDS-polyacrylamide gel electrophoresis of a protein obtained in Example 22.
[FIG. 8] FIG. 8 is a photograph showing the results of SDS-polyacrylamide gel electrophoresis of proteins obtained in Examples 23-26.
[FIG. 9] FIG. 9 is a photograph showing the results of SDS-polyacrylamide gel electrophoresis of a protein obtained in Example 28.
[FIG. 10] FIG. 10 is a photograph showing the results of SDS-polyacrylamide gel electrophoresis of a protein obtained in Comparative Example 1.
[FIG. 11] FIG. 11A is a photograph showing the results of SDS-polyacrylamide gel electrophoresis of a protein obtained in Example 32, and FIG. 11B is a photograph showing the results of SDS-polyacrylamide gel electrophoresis of a protein obtained in Example 33.

### Description of the Invention

In the present invention, the hydropathy index of the protein is calculated as follows. First, the total sum of the hydropathy indices of amino acids present in the protein is calculated. Next, the total sum is divided by the number of amino acid residues present in the protein so as to calculate the average value. The average value calculated is defined as the hydropathy index of the protein. Incidentally, known hydropathy indices are used as the hydropathy indices of respective amino acids (Hydropathy index: Kyte, J. & Doolittle, R. F. (1982) A simple method for displaying the hydropathic character of a protein. J. Mol. Biol. 157, 105-132). Specifically, Table 1 below shows hydropathy indices (hereinafter, also referred to as HI) of respective amino acids.

**[Table 1]**

| Amino acid | HI | Amino acid | HI |
|---|---|---|---|
| Alanine (Ala) | 1.8 | Leucine (Leu) | 3.8 |
| Arginine (Arg) | -4.5 | Lysine (Lys) | -3.9 |
| Asparagine (Asn) | -3.5 | Methionine (Met) | 1.9 |
| Aspartic acid (Asp) | -3.5 | Phenylalanine (Phe) | 2.8 |
| Cysteine (Cys) | 2.5 | Proline (Pro) | -1.6 |
| Glutamine (Gln) | -3.5 | Serine (Ser) | -0.8 |
| Glutamic acid (Glu) | -3.5 | Threonine (Thr) | -0.7 |
| Glycine (Gly) | -0.4 | Tryptophan (Trp) | -0.9 |
| Histidine (His) | -3.2 | Tyrosine (Tyr) | -1.3 |
| Isoleucine (Ile) | 4.5 | Valine (Val) | 4.2 |

The present inventors have found out that, by adding an aprotic polar solvent to a host expressing a hydrophilic recombinant protein having a hydropathy index of 0 or less to dissolve the host cell and adding an aqueous solvent such as water to the obtained host cell lysate to dilute the lysate, the hydrophilic recombinant protein having a hydropathy index of 0 or less can be collected in the supernatant. As a result, the present inventors have reached the present invention. In other words, the present inventors have found out that: by dissolving a host cell using an aprotic polar solvent, a lysate is obtained in which a hydrophilic recombinant protein having a hydropathy index of 0 or less is dissolved in the aprotic polar solvent; and by adding an aqueous solvent such as water to the lysate, the hydrophilic recombinant protein having a hydropathy index of 0 or less does not aggregate but most proteins other than the hydrophilic recombinant protein having a hydropathy index of 0 or less aggregate. As a result, the present inventors have reached the present invention. In the following, the hydrophilic protein refers to a hydrophilic protein having a hydropathy index of 0 or less unless otherwise specified. Further, in the following, the recombinant protein refers to a hydrophilic recombinant protein having a hydropathy index of 0 or less unless otherwise specified.

Although the aprotic polar solvent is not limited particularly, the purity of the aprotic polar solvent is preferably 90% or more, and more preferably 95% or more in terms of reducing contaminants. Preferably, the aprotic polar solvent is an aprotic polar solvent not having a cyclic structure. The use of the aprotic polar solvent not having a cyclic structure allows extraction of hydrophilic recombinant proteins such as a recombinant spider silk protein, a recombinant resilin and a recombinant collagen with higher purity. Examples of the aprotic polar solvent not having a cyclic structure include dimethyl sulfoxide (DMSO), N,N-dimethylformamide (DMF), and N,N-dimethylacetamide (DMA).

The aprotic polar solvent has a dipole moment of 3.0 D or more. The dipole moment is used as an index indicating the strength of the molecular polarity. The strength of the polarity increases with the dipole moment. Generally, the molecule having a dipole moment of 3.0 D or more is considered to have strong polarity. In the case of dissolving a compound having a polarity such as protein, a solvent having high polarity is effective. Table 2 below shows dipole moments of various organic compounds (organic solvents) based on Solvent Handbook (2007) published by Kodansha Scientific Ltd. Examples of the aprotic polar solvent having a dipole moment of 3.0 D or more include dimethyl sulfoxide (DMSO), N,N-dimethylformamide (DMF), N,N-dimethylacetamide (DMA), 1,3-dimethyl-2-imidazolidone (DMI), N-methyl-2-pyrrolidone (NMP), acetonitrile, etc.

**[Table 2]**

| Organic solvent | Dipole moment (D) | Organic solvent | Dipole moment (D) |
|---|---|---|---|
| DMSO | 4.3 | NMP | 4.09 |
| DMF | 3.86 | Acetonitrile | 3.44 |
| DMA | 3.72 | Acetone | 2.69 |
| DMI | 4.05 | Isopropanol | 1.68 |

In terms of extracting the recombinant protein with high purity, more preferably, the aprotic polar solvent has a dipole moment of 3.0 D or more, and does not have a cyclic structure. Further preferably, the aprotic polar solvent is one or more kinds of aprotic polar solvent selected from the group consisting of dimethyl sulfoxide (DMSO), N,N-dimethylformamide (DMF), and N,N-dimethylacetamide (DMA).

Preferably, the dissolving solvent is a solvent obtained by adding an inorganic salt to the aprotic polar solvent. This is preferred because, by adding an inorganic salt to the aprotic polar solvent, it becomes possible to obtain hydrophilic recombinant proteins such as a recombinant spider silk protein, a recombinant resilin and a recombinant collagen with higher purity. Although the inorganic salt is not limited particularly, preferably, it is at least one selected from the group consisting of alkali metal halides, alkaline-earth metal halides, alkaline-earth metal nitrate, and thiocyanate in terms of increasing the purity of the recombinant protein. Preferable examples of the alkali metal halides include LiCl and LiBr. Preferable examples of the alkaline-earth metal halides include CaCl₂, etc. Preferable examples of the alkaline-earth metal nitrate include Ca(NO₃)₂, etc. Preferable examples of the thiocyanate include NaSCN, etc.

When the dissolving solvent is the solvent obtained by adding an inorganic salt to the aprotic polar solvent, the concentration of the inorganic salt is preferably, but is not particularly limited to, 0.1 M or more, more preferably 0.25 M or more, and further preferably 0.5 M or more in terms of increasing the purity of the recombinant protein. Further, the upper limit of the concentration of the inorganic salt in the dissolving solvent is preferably, but is not particularly limited to, saturation or lower, e.g., 2.5 M or lower.

The addition amount of the dissolving solvent is not limited particularly as long as it allows dissolution of the host. However, in terms of increasing dissolubility, the addition amount of the dissolving solvent is preferably 0.5 times or more, more preferably 0.75 times or more, and further preferably 1 time or more the mass of the host (cell) by volume (mL)/mass (g) ratio. Further, in terms of operability, the addition amount of the dissolving solvent is preferably 10 times or less, more preferably 6 times or less, and further preferably 4 times or less the mass of the host by volume (mL)/mass (g) ratio. The host (cell) may be either in a dried state or in a wet state.

The lysate acquisition step in which the dissolving solvent is added to the host so as to obtain a host cell lysate is not limited particularly. However, in terms of solubility of the recombinant protein, the step is performed preferably at 20°C or higher, more preferably at 45°C or higher, and further preferably at 50°C or higher. Further, in terms of suppressing decomposition of the recombinant protein, the lysate acquisition step is performed preferably at 100°C or lower, and more preferably at 95°C or lower. Further, although the lysate acquisition step is not limited particularly, the step is performed preferably for 10 to 300 minutes, and more preferably for 30 to 180 minutes, for example. Insoluble substances may be separated before addition of the diluting solvent to the host cell lysate obtained in the lysate acquisition step. The separation of insoluble substances is not limited particularly as long as sediments (aggregates) can be separated. In terms of handleability, separation by filtration and/or separation by centrifugation are preferred. The separation by filtration may be performed using, e.g., a filter paper, a filtration membrane, etc. The conditions of the centrifugation are not limited particularly. For example, the centrifugation may be performed at room temperature (20°C±5°C), 8,000 x g to 15,000 x g for 5 to 20 minutes. The separation of insoluble substances may be performed two or more times.

The diluting solvent is an aqueous solvent. In the present invention, the aqueous solvent refers to a solvent containing water. Examples of the aqueous solvent include water, a mixture of water and a water-miscible organic solvent, a water-soluble buffer solution, an acid aqueous solution, and a basic aqueous solution. Examples of the acid aqueous solution include acid aqueous solutions such as formic acid, acetic acid and diluted hydrochloric acid. Examples of the basic aqueous solution include basic aqueous solutions such as a sodium hydroxide aqueous solution and a calcium hydroxide aqueous solution. Any known water-miscible organic solvent can be used as long as it is an organic solvent that can be mixed with water. Specific examples of the water-miscible organic solvent include alcohols such as methanol and ethanol.

In terms of obtaining the recombinant protein with high purity and convenience, preferably, the diluting solvent is water. Although the water is not limited particularly, preferably, it is pure water, distilled water, ultrapure water or the like in terms of reducing contaminants. In terms of further increasing the purity of the recombinant protein such as a recombinant spider silk protein, preferably, the diluting solvent is a mixture of water and ethanol. In terms of obtaining the recombinant protein with high purity in the supernatant containing the recombinant protein such as a recombinant spider silk protein, the addition amount of ethanol with respect to the whole mass of the dilution (the total mass of the host cell lysate and the diluting solvent) is preferably 5 mass% or more, more preferably 10 mass% or more, further preferably 15 mass% or more, and particularly preferably 25 mass% or more. Further, in terms of avoiding aggregation of the recombinant protein, the addition amount of ethanol is preferably 50 mass% or less, more preferably 45 mass% or less, and further preferably 40 mass% or less.

The diluting solvent may be a water-soluble buffer solution. Examples of the water-soluble buffer solution include a phosphoric acid-based buffer solution at pH 3 to 9 and a Tris-based buffer solution at pH 3 to 9. The concentration of the phosphoric acid-based buffer solution is, e.g., 10 to 20 mM. The concentration of the Tris-based buffer solution is, e.g., 10 to 100 mM. An example of the Tris-based buffer solution is a 50 mM Tris buffer solution at pH 3 to 9. An example of the phosphoric acid-based buffer solution is a 50 mM sodium phosphate buffer solution at pH 3 to 9.

By adding the diluting solvent to the host cell lysate for diluting the lysate, most of the contaminant proteins other than the recombinant protein such as a recombinant spider silk protein aggregate and the aggregated insoluble substances are separated and removed from the dilution, whereby a protein solution (supernatant) containing the recombinant protein is collected.

The addition amount of the diluting solvent is not limited particularly as long as it allows proteins other than the recombinant protein (in the following, also referred to as other proteins) to aggregate. In terms of uniform dilution, the addition amount of the diluting solvent is preferably 0.5 times or more, more preferably 0.75 times or more, and further preferably 1 time or more the amount of the host cell lysate by volume ratio. Further, in terms of avoiding aggregation of the recombinant protein, the addition amount of the diluting solvent is preferably 10 times or less, more preferably 6 times or less, and further preferably 4 times or less the amount of the host cell lysate.

In the above description, the separation of insoluble substances is not limited particularly as long as sediments (aggregates) can be separated. In terms of handleability, separation by filtration and/or separation by centrifugation are preferred. The separation by filtration may be performed using, e.g., a filter paper, a filtration membrane, etc. The conditions of the centrifugation are not limited particularly. For example, the centrifugation may be performed at room temperature (20°C±5°C), 8,000 x g to 15,000 x g for 5 to 20 minutes. The separation of insoluble substances may be performed two or more times.

The recombinant protein is not limited particularly as long as it is a hydrophilic recombinant protein having a hydropathy index (HI) of 0 or less. In terms of high extraction efficiency, the recombinant protein has HI of preferably -0.1 or less, and more preferably -0.3 or less.

Although the recombinant protein is not limited particularly, preferably, it is a protein selected from the group consisting of a recombinant spider silk protein, a recombinant resilin, and a recombinant collagen, for example.

The recombinant spider silk protein is not limited particularly as long as it is a recombinant spider silk protein derived from natural type spider silk proteins. Examples of the recombinant spider silk protein include variants, analogs, derivatives and the like of natural type spider silk proteins. In terms of excellent tenacity, the recombinant spider silk protein preferably is a recombinant spider silk protein derived from major dragline silk proteins produced in major ampullate glands of spiders. Examples of the major dragline silk proteins include major ampullate spidroins MaSp1 and MaSp2 derived from Nephila clavipes, and ADF3 and ADF4 derived from Araneus diadematus, etc. Further, the recombinant spider silk protein may be a recombinant spider silk protein derived from minor dragline silk produced in minor ampullate glands of spiders. Examples of the minor dragline silk proteins include minor ampullate spidroins MiSp1 and MiSp2 derived from Nephila clavipes. Further, the recombinant spider silk protein may be a recombinant spider silk protein derived from flagelliform silk proteins produced in flagelliform glands of spiders. Examples of the flagelliform silk proteins include flagelliform silk proteins derived from Nephila clavipes, etc.

Examples of the recombinant spider silk protein derived from major dragline silk proteins include a polypeptide containing two or more units of an amino acid sequence represented by the formula 1: REP1-REP2, preferably a polypeptide containing four or more units thereof, and more preferably a polypeptide containing six or more units thereof. In the recombinant spider silk protein derived from major dragline silk proteins, units of the amino acid sequence represented by the formula 1: REP1-REP2 may be the same or different from each other.

In the formula 1, the REP1 represents polyalanine. In the REP1, the number of alanine residues arranged in succession is preferably 2 or more, more preferably 3 or more, further preferably 4 or more, and particularly preferably 5 or more. Further, in the REP1, the number of alanine residues arranged in succession is preferably 20 or less, more preferably 16 or less, further preferably 14 or less, and particularly preferably 12 or less. In the formula 1, the REP2 is an amino acid sequence composed of 10 to 200 amino acid residues. The total number of glycine, serine, glutamine, proline and alanine residues contained in the amino acid sequence is 40% or more, preferably 50% or more, and more preferably 60% or more with respect to the total number of amino acid residues contained therein.

In the major dragline silk, the REP1 corresponds to a crystal region in a fiber where a crystal β sheet is formed, and the REP2 corresponds to an amorphous region in a fiber where most of the parts lack regular structures and that has more flexibility. Further, the [REP1-REP2] corresponds to a repetitious region (repetitive sequence) composed of the crystal region and the amorphous region, which is a characteristic sequence of dragline silk proteins.

An example of the polypeptide containing two or more units of the amino acid sequence represented by the formula 1: REP1-REP2 is a recombinant spider silk protein derived from ADF3 having an amino acid sequence represented by SEQ ID NO: 1. The amino acid sequence represented by SEQ ID NO: 1 is an amino acid sequence from the 1st residue to the 136th residue of an amino acid sequence that is obtained by adding an amino acid sequence (SEQ ID NO: 3) composed of a start codon, an His 10-tag and an HRV3C Protease (Human rhinovirus 3C Protease) recognition site to the N-terminal of a partial amino acid sequence of ADF3 obtained from the NCBI database (NCBI Genebank Accession No.: AAC47010, GI: 1263287). A protein ADF3Kai-small-M having the amino acid sequence represented by SEQ ID NO: 1 has HI of -0.948. Further, the polypeptide containing two or more units of the amino acid sequence represented by the formula 1: REP1-REP2 may be a polypeptide that is composed of an amino acid sequence represented by SEQ ID NO: 1 in which one or a plurality of amino acids have been substituted, deleted, inserted and/or added, and that has repetitious regions composed of crystal regions and amorphous regions and has HI of 0 or less.

In the present invention, "one or a plurality of' refers to 1 to 40, 1 to 35, 1 to 30, 1 to 25, 1 to 20, 1 to 15, 1 to 10, or 1 or a few, for example. Further, in the present invention, "one or a few" refers to 1 to 9, 1 to 8, 1 to 7, 1 to 6, 1 to 5, 1 to 4, 1 to 3, 1 to 2, or 1.

Further, an example of the polypeptide containing two or more units of the amino acid sequence represented by the formula 1: REP1-REP2 is a recombinant spider silk protein derived from ADF3 having an amino acid sequence represented by SEQ ID NO: 2. The amino acid sequence represented by SEQ ID NO: 2 is an amino acid sequence from the 1st residue to the 542nd residue of an amino acid sequence that is obtained by adding the amino acid sequence (SEQ ID NO: 3) composed of a start codon, an His 10-tag and an HRV3C Protease (Human rhinovirus 3C Protease) recognition site to the N-terminal of a partial amino acid sequence of ADF3 obtained from the NCBI database (NCBI Genebank Accession No.: AAC47010, GI: 1263287). A protein ADF3Kai-NN having the amino acid sequence represented by SEQ ID NO: 2 has HI of -0.92. Further, the polypeptide containing two or more units of the amino acid sequence represented by the formula 1: REP1-REP2 may be a polypeptide that is composed of an amino acid sequence represented by SEQ ID NO: 2 in which one or a plurality of amino acids have been substituted, deleted, inserted and/or added, and that has repetitious regions composed of crystal regions and amorphous regions and has HI of 0 or less.

Further, an example of the polypeptide containing two or more units of the amino acid sequence represented by the formula 1: REP1-REP2 is a recombinant spider silk protein derived from ADF4 having an amino acid sequence represented by SEQ ID NO: 6. The amino acid sequence represented by SEQ ID NO: 6 is an amino acid sequence from the 1st residue to the 318th residue of an amino acid sequence that is obtained by adding the amino acid sequence (SEQ ID NO: 3) composed of a start codon, an His 10-tag and an HRV3C Protease (Human rhinovirus 3C Protease) recognition site to the N-terminal of a partial amino acid sequence of ADF4 obtained from the NCBI database (NCBI Genebank Accession No.: AAC47011, GI: 1263289). A protein ADF4Kai-W having the amino acid sequence represented by SEQ ID NO: 6 has HI of -0.357. Further, the polypeptide containing two or more units of the amino acid sequence represented by the formula 1: REP1-REP2 may be a polypeptide that is composed of an amino acid sequence represented by SEQ ID NO: 6 in which one or a plurality of amino acids have been substituted, deleted, inserted and/or added, and that has repetitious regions composed of crystal regions and amorphous regions and has HI of 0 or less.

An example of the recombinant spider silk protein derived from minor dragline silk proteins is a polypeptide containing an amino acid sequence represented by the formula 2: (Gly-Gly-Z)m(Gly-Ala)l(Ala)o.

In the formula 2, Z indicates any one of amino acids, and in particular, it preferably is an amino acid selected from the group consisting of Ala, Tyr and Gln. Preferably, m is an integer in a range of 1 to 4, l is an integer in a range of 0 to 4, and o is an integer in a range of 1 to 6.

Among spider silks, the minor dragline silk is wound spirally from the center of a spider net, and used as a reinforcement of the net and a yarn to wrap a captured prey. The minor dragline silk is inferior to the major dragline silk in tensile strength, but is known to have high stretchability. The reason for this is considered to be as follows: in the minor dragline silk, since many crystal regions are formed of regions where glycine and alanine are arranged alternately in succession, hydrogen bonds in the crystal regions weaken easily as compared with the major dragline silk whose crystal regions are formed only of alanine.

An example of the recombinant spider silk protein derived from flagelliform silk proteins is a polypeptide containing an amino acid sequence represented by the formula 3: (Gly-Pro-Gly-Gly-X)n. Units of the amino acid sequence represented by Gly-Pro-Gly-Gly-X may be the same or different from each other.

In the formula 3, X indicates any one of amino acids, and in particular, it preferably is an amino acid selected from the group consisting of Ala, Ser, Tyr and Val. Further, n is an integer of 4 or larger, preferably an integer of 10 or larger, and more preferably an integer of 20 or larger.

An example of the polypeptide containing the amino acid sequence represented by the formula 3: (Gly-Pro-Gly-Gly-X)n is a recombinant spider silk protein derived from flagelliform silk proteins having an amino acid sequence represented by SEQ ID NO: 8. The amino acid sequence represented by SEQ ID NO: 8 is an amino acid sequence obtained by adding the amino acid sequence (SEQ ID NO: 3) composed of a start codon, an His 10-tag and an HRV3C Protease recognition site to the N-terminal of a partial sequence of flagelliform silk protein of Nephila clavipes obtained from the NCBI database (NCBI Genebank Accession No.: AAF36090, GI: 7106224), specifically, an amino acid sequence thereof from the 1220th residue to the 1662nd residue that corresponds to repetitive sections and motifs in said partial sequence of flagelliform silk protein. A protein Flag92-short2-UU having the amino acid sequence represented by SEQ ID NO: 8 has HI of -0.482. Further, the polypeptide containing the amino acid sequence represented by the formula 3: (Gly-Pro-Gly-Gly-X)n may be a polypeptide that is composed of an amino acid sequence represented by SEQ ID NO: 8 in which one or a plurality of amino acids have been substituted, deleted, inserted and/or added, and that has repetitious regions composed of amorphous regions and has HI of 0 or less.

Among spider silks, the flagelliform silk does not have crystal regions but has repetitious regions composed of amorphous regions, which is a major characteristic of the flagelliform silk. It is considered that since the major dragline silk and the like have repetitious regions composed of crystal regions and amorphous regions, they have both of high strength and stretchability. Meanwhile, regarding the flagelliform silk, the strength is inferior to that of the major dragline silk but the stretchability is high. The reason for this is considered to be that the flagelliform silk is composed mostly of amorphous regions.

The recombinant spider silk protein preferably is a recombinant spider silk protein derived from ADF3, which is one of two principal dragline silk proteins of Araneus diadematus. The recombinant spider silk protein derived from ADF3 has advantages of basically having high strength elongation and toughness and being synthesized easily.

An example of the recombinant resilin may be a recombinant resilin derived from natural resilin. Examples of the recombinant resilin derived from natural resilin include a polypeptide containing two or more units of an amino acid sequence represented by the formula 4: REP3, preferably a polypeptide containing 4 or more units thereof, more preferably a polypeptide containing 10 or more units thereof, and further preferably a polypeptide containing 20 or more units thereof. Units of the amino acid sequence represented by REP3 may be the same or different from each other.

In the formula 4, REP3 indicates an amino acid sequence composed of (Ser-J-J-Tyr-Gly-U-Pro). J indicates any one of amino acids, and in particular, it preferably is an amino acid selected from the group consisting of Asp, Ser and Thr. Further, U indicates any one of amino acids, in particular, it preferably is an amino acid selected from the group consisting of Pro, Ala, Thr and Ser.

An example of the polypeptide containing two or more units of the amino acid sequence represented by the formula 4: REP3 is a recombinant resilin having an amino acid sequence represented by SEQ ID NO: 10. The amino acid sequence represented by SEQ ID NO: 10 is an amino acid sequence obtained as follows: in a partial amino acid sequence of resilin obtained from the NCBI web database (NCBI Genebank Accession No.: NP_611157, GI: 24654243), the 87th residue Thr and the 95th residue Asn are substituted with Ser and Asp, respectively, and the substituted sequence is cut out from the 19th residue to the 321st residue, and then an amino acid sequence (SEQ ID NO: 12) composed of a start codon and an His 6-tag is added to the N-terminal of the sequence cut out. The recombinant resilin having the amino acid sequence represented by SEQ ID NO: 10 has HI of -1.229. Further, the polypeptide containing two or more units of the amino acid sequence represented by the formula 4: REP3 may be a polypeptide that is composed of an amino acid sequence represented by SEQ ID NO: 10 in which one or a plurality of amino acids have been substituted, deleted, inserted and/or added, and that has HI of 0 or less. Note that the amino acid sequence represented by SEQ ID NO: 10 is the same as the amino acid sequence of rec1-resilin described in the following known document: "Synthesis and properties of crosslinked recombinant pro-resilin" [Christopher M. Elvin, Andrew G. Carr, Mickey G. Huson, Jane M. Maxwell, Roger D. Pearson, Tony Vuocolo, Nancy E. Liyou, Darren C. C. Wong, David J. Merritt & Nicholas E. Dixon, Nature 437, 999-1002(13 October 2005)].

Further, an example of the polypeptide containing two or more units of the amino acid sequence represented by the formula 4: REP3 is a recombinant resilin having an amino acid sequence represented by SEQ ID NO: 13. The amino acid sequence represented by SEQ ID NO: 13 is an amino acid sequence obtained by adding the amino acid sequence (SEQ ID NO: 12) composed of a start codon and an His 6-tag to the N-terminal of a partial sequence of resilin of Drosophila melanogaster obtained from the NCBI web database (NCBI Genebank Accession No.: Q9V7U0.1, GI: 75026432), specifically, an amino acid sequence thereof from the 19th residue to the 321st residue that corresponds to repetitive sections and motifs in said partial sequence of resilin. The recombinant resilin having the amino acid sequence represented by SEQ ID NO: 13 has HI of -1.229. Further, the polypeptide containing two or more units of the amino acid sequence represented by the formula 4: REP3 may be a polypeptide that is composed of an amino acid sequence represented by SEQ ID NO: 13 in which one or a plurality of amino acids have been substituted, deleted, inserted and/or added, and that has HI of 0 or less.

Resilin is a rubber-like protein present in a specific region of the insect cuticle, and is an excellent elastic material. This substance has an elasticity of 97% and loses only 3% of storage energy as heat. It is considered that cross-linking between tyrosines in resilin brings this elasticity. The dityrosine-crosslinked resilin imparts excellent elasticity to the cuticle, and plays an important role in insect's flight system and jumping system.

An example of the recombinant collagen may be a recombinant collagen derived from natural collagens. Examples of the recombinant collagen derived from natural collagens include a polypeptide containing, in succession, 5 or more units of the amino acid sequence represented by the formula 5: REP4, preferably a polypeptide containing 8 or more units thereof, and more preferably a polypeptide containing 10 or more units thereof.

In the formula 5, REP4 indicates an amino acid sequence composed of Gly-X-Y. X and Y respectively indicate any one of amino acids except Gly. In the formula 5, units of the amino acid sequence represented by REP4 may be the same or different from each other.

An example of the polypeptide containing, in succession, 10 or more units of the amino acid sequence represented by the formula 5: REP4 is a recombinant collagen having an amino acid sequence represented by SEQ ID NO: 16. The amino acid sequence represented by SEQ ID NO: 16 is an amino acid sequence obtained by adding the amino acid sequence (SEQ ID NO: 15) composed of a start codon, an His 6-tag and Ser-Ser-Gly-Ser-Ser to the N-terminal of a partial sequence of human collagen type 4 obtained from the NCBI database (NCBI Genebank Accession No.: CAA56335.1, GI: 3702452), specifically, an amino acid sequence thereof from the 301st residue to the 540th residue that corresponds to repetitive sections and motifs in said partial sequence of human collagen type 4. Further, the polypeptide containing, in succession, 10 or more units of the amino acid sequence represented by the formula 5: REP5 may be a polypeptide that is composed of an amino acid sequence represented by SEQ ID NO: 16 in which one or a plurality of amino acids have been substituted, deleted, inserted and/or added.

Collagen is one of the proteins constituting the dermis, ligament, tendon, bone, cartilage, etc., and is the main component of extracellular matrices of multicellular animals. 30 kinds or more of human collagens have been reported. All of these collagens have a repetitious amino acid sequence represented by REP4 called a collagen-like sequence, which is a characteristic sequence of human collagens.

The recombinant protein can be produced using a host that has been transformed by an expression vector containing a gene encoding a recombinant protein. A method for producing a gene is not limited particularly, and it may be produced by amplifying a gene encoding a recombinant protein from a cell containing the desired gene by a polymerase chain reaction (PCR), etc., and cloning it, or may be synthesized chemically. A method for chemically synthesizing a gene also is not limited particularly, and it can be synthesized as follows, for example: based on information of an amino acid sequence of a recombinant protein, oligonucleotides that have been synthesized automatically with AKTA oligopilot plus 10/100 (GE Healthcare Japan Corporation) are linked by PCR, etc. At this time, in order to facilitate purification and observation of protein, a gene may be synthesized that encodes a protein composed of an amino acid sequence in which an amino acid sequence composed of an His tag has been added to the N-terminal of the amino acid sequence of the target protein.

Examples of the expression vector include a plasmid, a phage, a virus and the like that can express protein based on a DNA sequence. The plasmid-type expression vector is not limited particularly as long as it allows a target gene to be expressed in a host cell and it can amplify itself. For example, in the case of using Escherichia coli Rosetta (DE3) as a host, a pET22b(+) plasmid vector, a pCold plasmid vector and the like can be used. Among these, in terms of productivity of protein, the use of the pET22b(+) plasmid vector is preferred. Examples of the host (cell) include animal cells, plant cells, microbes and the like that can be used as protein expression systems. In terms of the production cost, the use of microbes is preferred. In terms of safety and operability, the use of Escherichia coli is more preferred.

When producing the recombinant protein using a microbe such as Escherichia coli as a host, the molecular weight of the recombinant protein is preferably 500 kDa or less, more preferably 300 kDa or less, and further preferably 200 kDa or less in terms of productivity.

In the supernatant containing the recombinant protein, the purity (purification degree) of the recombinant protein with respect to the total proteins is preferably 40 mass% or more, more preferably 45 mass% or more, further preferably 50 mass% or more, further more preferably 60 mass% or more, and particularly preferably 65 mass% or more. In the present invention, the purity can be measured by staining the polyacrylamide gel after SDS-PAGE with Coomassie Brilliant Blue (CBB) or Oriole Fluorescent Gel Stain and analyzing electrophoresis images after staining using analysis software ImageLab (Bio-RAD Laboratories, Inc.).

The supernatant containing the recombinant protein can be purified further by dialysis, etc. The recombinant protein may be aggregated by adding ethanol to the supernatant containing the recombinant protein so that the final concentration would be 50 mass% or more, preferably 70 mass% or more, and more preferably 90 mass% or more. Further, the supernatant containing the recombinant protein may be powdered. A method for powdering the protein is not limited particularly. The recombinant protein may be powdered by freeze-drying the recombinant protein aggregated by ethanol, or may be powdered directly by spray-drying the supernatant containing the recombinant protein. The obtained powder of the recombinant spider silk protein can be used for wet spinning, formation of cast films, and the like. The obtained powders of the recombinant resilin and the recombinant collagen can be used for formation of gels, cast films, porous bodies, and the like.

### Examples

Hereinafter, the present invention will be described in further detail by way of examples. Note that the present invention is not limited to the following examples.

### (Expression of recombinant protein in Escherichia coli)

### <Gene Synthesis>

### (1) Gene Synthesis ofADF3Kai-small-M

A partial amino acid sequence of ADF3, which is one of two principal dragline silk proteins of Araneus diadematus, was obtained from the NCBI web database (NCBI Genebank Accession No.: AAC47010, GI: 1263287), and an amino acid sequence (SEQ ID NO: 3) composed of a start codon, an His 10-tag and an HRV3C Protease (Human rhinovirus 3C Protease) recognition site was added to the N-terminal of the partial amino acid sequence of ADF3, so as to synthesize a gene encoding a polypeptide composed of an amino acid sequence (SEQ ID NO: 1), i.e., the 1st residue to the 136th residue of the resultant sequence. Consequently, a pUC57 vector to which a gene of ADF3Kai-small-M composed of a base sequence represented by SEQ ID NO: 4 had been introduced was obtained (having an Nde I site immediately upstream of the 5' terminal of the gene and an Xba I site immediately downstream of the 5' terminal thereof). Thereafter, the gene was subjected to a restriction enzyme treatment with Nde I and EcoR I, and recombined into a pET22b(+) expression vector. Thus, a pET22b(+) vector to which the gene ofADF3Kai-small-M had been introduced was obtained.

### (2) Gene Synthesis of ADF3Kai-NN

A partial amino acid sequence of ADF3, which is one of two principal dragline silk proteins of Araneus diadematus, was obtained from the NCBI web database (NCBI Genebank Accession No.: AAC47010, GI: 1263287), and the amino acid sequence (SEQ ID NO: 3) composed of a start codon, an His 10-tag and an HRV3C Protease (Human rhinovirus 3C Protease) recognition site was added to the N-terminal of the partial amino acid sequence of ADF3, so as to synthesize a gene encoding a polypeptide composed of an amino acid sequence (SEQ ID NO: 2), i.e., the 1st residue to the 542nd residue of the resultant sequence. Consequently, a pUC57 vector to which a gene of ADF3Kai-NN composed of a base sequence represented by SEQ ID NO: 5 had been introduced was obtained (having an Nde I site immediately upstream of the 5' terminal of the gene and an Xba I site immediately downstream of the 5' terminal thereof). Thereafter, the gene was subjected to a restriction enzyme treatment with Nde I and EcoR I, and recombined into a pET22b(+) expression vector. Thus, a pET22b(+) vector to which the gene of ADF3Kai-NN had been introduced was obtained.

### (3) Gene Synthesis of ADF4Kai-W

A partial amino acid sequence of ADF4, which is the other principal dragline silk protein of Araneus diadematus, was obtained from the NCBI web database (NCBI Genebank Accession No.: AAC47011, GI: 1263289), and the amino acid sequence (SEQ ID NO: 3) composed of a start codon, an His 10-tag and an HRV3C Protease (Human rhinovirus 3C Protease) recognition site was added to the N-terminal of the partial amino acid sequence of ADF4, so as to synthesize a gene encoding a polypeptide composed of an amino acid sequence (SEQ ID NO: 6), i.e., the 1st residue to the 318th residue of the resultant sequence. Consequently, a pUC57 vector to which a gene of ADF4Kai-W composed of a base sequence represented by SEQ ID NO: 7 had been introduced was obtained (having an Nde I site immediately upstream of the 5' terminal of the gene and an Xba I site immediately downstream of the 5' terminal thereof). Thereafter, the gene was subjected to a restriction enzyme treatment with Nde I and EcoR I, and recombined into a pET22b(+) expression vector. Thus, a pET22b(+) vector to which the gene of ADF4Kai-W had been introduced was obtained.

### (4) Gene Synthesis of Flag92-short2-UU

A partial sequence of flagelliform silk protein of Nephila clavipes was obtained from the NCBI web database (NCBI Genebank Accession No.: AAF36090, GI: 7106224), and a gene encoding a polypeptide composed of an amino acid sequence (SEQ ID NO: 8) was synthesized. The amino acid sequence (SEQ ID NO: 8) is an amino acid sequence obtained by adding the amino acid sequence (SEQ ID NO: 3) composed of a start codon, an His 10-tag and an HRV3C Protease recognition site to the N-terminal of the partial amino acid sequence of flagelliform silk protein from the 1220th residue to the 1662nd residue that corresponds to repetitive sections and motifs in said sequence. Consequently, a pUC57 vector to which a gene of Flag92-short2-UU composed of a base sequence represented by SEQ ID NO: 9 had been introduced was obtained (having an Nde I site immediately upstream of the 5' terminal of the gene and an Xba I site immediately downstream of the 5' terminal thereof). Thereafter, the gene was subjected to a restriction enzyme treatment with Nde I and EcoR I, and recombined into a pET22b(+) expression vector. Thus, a pET22b(+) vector to which the gene of Flag92-short2-UU had been introduced was obtained.

### (5) Gene Synthesis of Resilin-Kai

A partial amino acid sequence of resilin was obtained from the NCBI web database (NCBI Genebank Accession No.: NP_611157, GI: 24654243), and a gene encoding a polypeptide (Resilin-Kai) composed of an amino acid sequence (SEQ ID NO: 10) was synthesized. The amino acid sequence (SEQ ID NO: 10) is an amino acid sequence obtained by: substituting the 87th residue Thr and the 95th residue Asn of the partial amino acid sequence of resilin with Ser and Asp, respectively; cutting out the substituted sequence from the 19th residue to the 321st residue; and adding an amino acid sequence (SEQ ID NO: 12) composed of a start codon and an His 6-tag to the N-terminal of the sequence cut out. Consequently, a pUC57 vector to which a gene of Resilin-Kai composed of a base sequence represented by SEQ ID NO: 11 had been introduced was obtained (having an Nde I site immediately upstream of the 5' terminal of the gene and an Xba I site immediately downstream of the 5' terminal thereof). Thereafter, the gene was subjected to a restriction enzyme treatment with Nde I and EcoR I, and recombined into a pET22b(+) expression vector. Thus, a pET22b(+) vector to which the gene of Resilin-Kai had been introduced was obtained.

### (6) Gene Synthesis of Drosophila-Resilin-Kai

A partial sequence of resilin of Drosophila melanogaster was obtained from the NCBI web database (NCBI Genebank Accession No.: Q9V7U0.1, GI: 75026432), and a gene encoding a polypeptide composed of an amino acid sequence (SEQ ID NO: 13) was synthesized. The amino acid sequence (SEQ ID NO: 13) is an amino acid sequence obtained by adding the amino acid sequence (SEQ ID NO: 12) composed of a start codon and an His 6-tag to the N-terminal of the partial amino acid sequence of resilin from the 19th residue to the 321st residue that corresponds to repetitive sections and motifs in said sequence. Consequently, a pUC57 vector to which a gene of Drosophila-Resilin-Kai composed of a base sequence represented by SEQ ID NO: 14 had been introduced was obtained (having an Nde I site immediately upstream of the 5' terminal of the gene and an Xba I site immediately downstream of the 5' terminal thereof). Thereafter, the gene was subjected to a restriction enzyme treatment with Nde I and EcoR I, and recombined into a pET22b(+) expression vector. Thus, a pET22b(+) vector to which the gene of Drosophila-Resilin-Kai had been introduced was obtained.

### (7) Gene Synthesis of Collagen-type4-Kai

A partial sequence of human collagen type 4 was obtained from the NCBI web database (NCBI Genebank Accession No.: CAA56335.1, GI: 3702452), and a gene encoding a polypeptide composed of an amino acid sequence (SEQ ID NO: 16) was synthesized. The amino acid sequence (SEQ ID NO: 16) is an amino acid sequence obtained by adding an amino acid sequence (SEQ ID NO: 15) composed of a start codon, an His 6-tag and Ser-Ser-Gly-Ser-Ser to the N-terminal of the partial amino acid sequence of human collagen type 4 from the 301st residue to the 540th residue that corresponds to repetitive sections and motifs in said sequence. Consequently, a pUC57 vector to which a gene of Collagen-type4-Kai composed of a base sequence represented by SEQ ID NO: 17 had been introduced was obtained (having an Nde I site immediately upstream of the 5' terminal of the gene and an Xba I site immediately downstream of the 5' terminal thereof). Thereafter, the gene was subjected to a restriction enzyme treatment with Nde I and EcoR I, and recombined into a pET22b(+) expression vector. Thus, a pET22b(+) vector to which the gene of Collagen-type4-Kai had been introduced was obtained.

### <Expression of Protein>

The vectors obtained, i.e., the pET22b(+) expression vector to which the gene of ADF3Kai-small-M had been introduced, the pET22b(+) expression vector A to which the gene of ADF3Kai-NN had been introduced, the pET22b(+) expression vector to which the gene of ADF4Kai-W had been introduced, the pET22b(+) vector to which the gene of Flag92-short2-UU had been introduced, the pET22b(+) vector to which the gene of Resilin-Kai had been introduced, the pET22b(+) vector to which the gene of Drosophila-Resilin-Kai had been introduced and the pET22b(+) vector to which the gene of Collagen-type4-Kai had been introduced, were each transformed into Escherichia coli Rosetta (DE3). Each of the obtained single colonies was incubated for 15 hours in 2 mL of an LB culture medium containing ampicillin. Thereafter, 1.4 ml of said culture solution was added to 140 mL of an LB culture medium containing ampicillin, and incubated to an OD₆₀₀ of 3.5 under the conditions of 37°C and 200 rpm. Next, the culture solution with the OD₆₀₀ of 3.5 was added to 7L of a 2xYT culture medium containing ampicillin, together with 140 mL of 50% glucose, and incubated further to the OD₆₀₀ of 4.0. Thereafter, isopropyl-β-thiogalactopyranoside (IPTG) was added to the obtained culture solution with the OD₆₀₀ of 4.0 so that the final concentration would be 0.5 mM, thereby inducing the expression of protein. After a lapse of two hours from the addition of IPTG, the culture solution was centrifuged and bacterial cells were collected. By using protein solutions prepared from the bacterial cells before the addition of IPTG and after the addition of IPTG, in accordance with a general protocol, the expression of target proteins with the addition of IPTG was confirmed by SDS-PAGE (ADF3Kai-small-M: about 12.5 kDa, ADF3Kai-NN: about 47.5 kDa, ADF4Kai-W: about 26.4 kDa, Flag92-short2-UU: about 36.9 kDa, Resilin-Kai: about 28.5 kDa, Drosophila-Resilin-Kai: about 28.5 kDa, Collagen-type4-Kai: about 24.5 kDa). The Escherichia coli expressing the protein ADF3Kai-small-M, the Escherichia coli expressing the protein ADF3Kai-NN, the Escherichia coli expressing the protein ADF4Kai-W, the Escherichia coli expressing the protein Flag92-short2-UU, the Escherichia coli expressing the protein Resilin-Kai, the Escherichia coli expressing the protein Drosophila-Resilin-Kai and the Escherichia coli expressing the protein Collagen-type4-Kai were stored in a freezer (-20°C).

Table 3 below indicates the hydropathy indices (HI) of the proteins ADF3Kai-small-M, ADF3Kai-NN, ADF4Kai-W, Flag92-short2-UU, Resilin-Kai, Drosophila-Resilin-Kai and Collagen-type4-Kai. All of these proteins ADF3Kai-small-M, ADF3Kai-NN, ADF4Kai-W, Flag92-short2-UU, Resilin-Kai, Drosophila-Resilin-Kai and Collagen-type4-Kai were hydrophilic proteins having HI of 0 or less.

**[Table 3]**

| Recombinant protein | HI |
|---|---|
| ADF3Kai-small-M | -0.948 |
| ADF3Kai-NN | -0.92 |
| ADF4Kai-W | -0.357 |
| Flag92-short2-UU | -0.482 |
| Resilin-Kai | -1.229 |
| Drosophila-Resilin-Kai | -1.229 |
| Collagen-type4-Kai | -0.792 |

### (Example 1)

(1) To about 0.1 g of dried bacterial cells of the Escherichia coli expressing the protein ADF3Kai-small-M, 1 ml of DMSO containing 1 M LiCl was added so as to disperse the bacterial cells, followed by standing at 60°C for 30 minutes. Thus, a lysate of the Escherichia coli was obtained.
(2) The lysate of the Escherichia coli obtained was diluted with an equivalent amount of ultrapure water, and the obtained dilution was centrifuged at 20°C, 11,000 x g for 5 minutes using a centrifuge ("MX-305" manufactured by TOMY SEIKO Co., Ltd.). Then, the supernatant containing the protein ADF3Kai-small-M was collected.

### (Example 2)

(1) To about 0.1 g of dried bacterial cells of the Escherichia coli expressing the protein ADF3Kai-NN, 1 mL of DMSO containing 0.5 M LiCl was added so as to disperse the bacterial cells, followed by standing at 60°C for 30 minutes. Thus, a lysate of the Escherichia coli was obtained.
(2) The lysate of the Escherichia coli obtained was diluted with an equivalent amount of ultrapure water, and the obtained dilution was centrifuged at 20°C, 11,000 x g for 5 minutes using a centrifuge ("MX-305" manufactured by TOMY SEIKO Co., Ltd.). Then, the supernatant containing the protein ADF3Kai-NN was collected.

### (Example 3)

The supernatant containing the protein ADF3Kai-NN was collected in the same manner as in Example 2 except that an equivalent amount of an ethanol aqueous solution at a concentration of 10 mass% (hereinafter, referred to as 10% ethanol) was used instead of ultrapure water to dilute the lysate of the Escherichia coli.

### (Example 4)

The supernatant containing the protein ADF3Kai-NN was collected in the same manner as in Example 2 except that an equivalent amount of an ethanol aqueous solution at a concentration of 30 mass% (hereinafter, referred to as 30% ethanol) was used instead of ultrapure water to dilute the lysate of the Escherichia coli.

### (Example 5)

The supernatant containing the protein ADF3Kai-NN was collected in the same manner as in Example 2 except that an equivalent amount of an ethanol aqueous solution at a concentration of 50 mass% (hereinafter, referred to as 50% ethanol) was used instead of ultrapure water to dilute the lysate of the Escherichia coli.

### (Example 6)

The supernatant containing the protein ADF3Kai-NN was collected in the same manner as in Example 2 except that an equivalent amount of an ethanol aqueous solution at a concentration of 70 mass% (hereinafter, referred to as 70% ethanol) was used instead of ultrapure water to dilute the lysate of the Escherichia coli.

### (Example 7)

The supernatant containing the protein ADF3Kai-NN was collected in the same manner as in Example 2 except that an equivalent amount of an ethanol aqueous solution at a concentration of 80 mass% (hereinafter, referred to as 80% ethanol) was used instead of ultrapure water to dilute the lysate of the Escherichia coli.

### (Example 8)

The supernatant containing the protein ADF3Kai-NN was collected in the same manner as in Example 2 except that an equivalent amount of an ethanol aqueous solution at a concentration of 90 mass% (hereinafter, referred to as 90% ethanol) was used instead of ultrapure water to dilute the lysate of the Escherichia coli.

### (Example 9)

(1) To about 0.1 g of dried bacterial cells of the Escherichia coli expressing the protein ADF3Kai-NN, 1 mL of DMSO containing 0.5 M LiCl was added so as to disperse the bacterial cells, followed by standing at 60°C for 30 minutes. Thus, a lysate of the Escherichia coli was obtained.
(2) The lysate of the Escherichia coli obtained was diluted with an equivalent amount of ultrapure water, and the obtained dilution was centrifuged at 20°C, 11,000 x g for 5 minutes using a centrifuge ("MX-305" manufactured by TOMY SEIKO Co., Ltd.). Then, the supernatant containing the protein ADF3Kai-NN was collected.

### (Example 10)

The supernatant containing the protein ADF3Kai-NN was collected in the same manner as in Example 9 except that an equivalent amount of a 50 mM Tris buffer solution (pH 3) was used instead of ultrapure water to dilute the lysate of the Escherichia coli.

### (Example 11)

The supernatant containing the protein ADF3Kai-NN was collected in the same manner as in Example 9 except that an equivalent amount of a 50 mM Tris buffer solution (pH 5) was used instead of ultrapure water to dilute the lysate of the Escherichia coli.

### (Example 12)

The supernatant containing the protein ADF3Kai-NN was collected in the same manner as in Example 9 except that an equivalent amount of a 50 mM Tris buffer solution (pH 7) was used instead of ultrapure water to dilute the lysate of the Escherichia coli.

### (Example 13)

The supernatant containing the protein ADF3Kai-NN was collected in the same manner as in Example 9 except that an equivalent amount of a 50 mM Tris buffer solution (pH 9) was used instead of ultrapure water to dilute the lysate of the Escherichia coli.

### (Example 14)

The supernatant containing the protein ADF3Kai-NN was collected in the same manner as in Example 2 except that, instead of the DMSO containing 0.5 M LiCl, 1 mL of DMSO containing 0.25 M LiCl was added to about 0.1 g of dried bacterial cells of the Escherichia coli expressing the protein ADF3Kai-NN.

### (Example 15)

The supernatant containing the protein ADF3Kai-NN was collected in the same manner as in Example 2 except that, instead of the DMSO containing 0.5 M LiCl, 1 mL of DMSO was added to about 0.1 g of dried bacterial cells of the Escherichia coli expressing the protein ADF3Kai-NN.

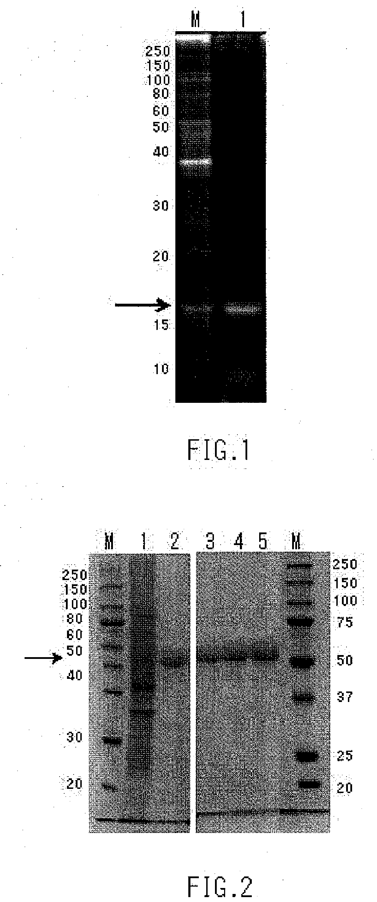

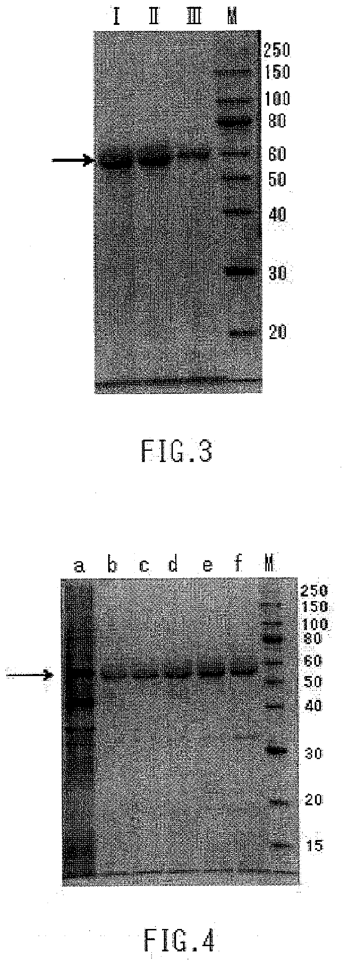

SDS-PAGE was performed using the supernatant containing the protein ADF3Kai-small-M obtained in Example 1 and the supernatants containing the protein ADF3Kai-NN obtained in Examples 2-15. Specifically, to 30 µL of the supernatant obtained in each example, 30 µL of a 2xSDS buffer solution (4 w/v% SDS, 20 w/v% glycerol, 10 w/v% 2-mercaptoethanol, 0.004 w/v% Bromophenol Blue, 0.125 M Tris, pH 6.8) was added, followed by heat treatment at 95°C for 10 minutes and cooling at room temperature. The heat-denatured protein solution obtained was subjected to polyacrylamide gel electrophoresis so as to check the band. After electrophoresis, the gel was stained with Oriole Fluorescent Gel Stain using the supernatant in Example 1, and the gels were stained with Coomassie Brilliant Blue (CBB) using the supernatants in the other examples. FIGS. 1-4 show the results. The purity of the target protein (recombinant spider silk protein) was calculated by analyzing images of electrophoresis photographs of the protein after staining using analysis software ImageLab (Bio-RAD Laboratories, Inc.). Table 4 below shows the results. For comparison, FIGS. 2 and 4 also show the results of SDS-PAGE using the lysate of the Escherichia coli in Example 2. In the electrophoresis, 1.5 µg of the protein was used for Oriole Fluorescent Gel Stain, and 7 µg of the protein was used for CBB.

**[Table 4]**

| | Dissolving solvent | Diluting solvent | Purity (%) |
|---|---|---|---|
| Example 1 | DMSO (containing 1 M LiCl) | Ultrapure water | 64.5 |
| Example 2 | DMSO (containing 0.5 M LiCl) | Ultrapure water | 64.5 |
| Example 3 | DMSO (containing 0.5 M LiCl) | 10% ethanol | 67.9 |
| Example 4 | DMSO (containing 0.5 M LiCl) | 30% ethanol | 70.1 |
| Example 5 | DMSO (containing 0.5 M LiCl) | 50% ethanol | 70.3 |
| Example 6 | DMSO (containing 0.5 M LiCl) | 70% ethanol | 64.9 |
| Example 7 | DMSO (containing 0.5 M LiCl) | 80% ethanol | 64.3 |
| Example 8 | DMSO (containing 0.5 M LiCl) | 90% ethanol | 40.1 |
| Example 9 | DMSO (containing 0.5 M LiCl) | Ultrapure water | 67.6 |
| Example 10 | DMSO (containing 0.5 M LiCl) | 50 mM Tris buffer solution (pH 3) | 65.8 |
| Example 11 | DMSO (containing 0.5 M LiCl) | 50 mM Tris buffer solution (pH 5) | 67.0 |
| Example 12 | DMSO (containing 0.5 M LiCl) | 50 mM Tris buffer solution (pH 7) | 61.9 |
| Example 13 | DMSO (containing 0.5 M LiCl) | 50 mM Tris buffer solution (pH 9) | 56.0 |
| Example 14 | DMSO (containing 0.25 M LiCl) | Ultrapure water | 55.6 |
| Example 15 | DMSO | Ultrapure water | 48.0 |

FIG. 1 shows the results of SDS-PAGE of the protein obtained in Example 1. In FIG. 1, a lane M indicates a molecular-weight marker, and a lane 1 indicates the protein contained in the supernatant obtained in Example 1. In FIG. 1, an arrow indicates the band of the protein corresponding to ADF3Kai-small-M (molecular weight: about 12.5 kDa). It was confirmed from FIG. 1 and Table 4 that the supernatant containing the target recombinant spider silk protein (ADF3Kai-small-M) was obtained by dissolving the Escherichia coli with the DMSO containing 1 M LiCl and diluting the lysate of the Escherichia coli with ultrapure water. In the proteins contained in the supernatant, the purity of the target recombinant spider silk protein (ADF3Kai-small-M) was 60% or more, which is a high purification rate.

FIG. 2 shows the results of SDS-PAGE of the proteins obtained in Examples 2-5. In FIG. 2, a lane M indicates a molecular-weight marker, a lane 1 indicates the protein contained in the lysate of the Escherichia coli of Example 2, and lanes 2, 3, 4 and 5 indicate the proteins contained in the supernatants obtained in Examples 2, 3, 4 and 5, respectively. FIG. 3 shows the results of SDS-PAGE of the proteins obtained in Examples 6-8. In FIG. 3, a lane M indicates a molecular-weight marker, and lanes I, II and III indicate the proteins contained in the supernatants obtained in Examples 6, 7 and 8, respectively. In FIGS. 2 and 3, each arrow indicates the band of the protein corresponding to ADF3Kai-NN (molecular weight: about 47.5 kDa). It was confirmed from FIGS. 2, 3 and Table 4 that the supernatants containing the target recombinant spider silk protein (ADF3Kai-NN) were obtained by dissolving the Escherichia coli with the DMSO containing 0.5 M LiCl and diluting the lysate of the Escherichia coli with various kind of aqueous solvents. It also was found that, when the mixture of water and ethanol was used as the diluting solvent and the concentration of the ethanol in the dilution (the mixture of the lysate of the Escherichia coli and the diluting solvent) was 35 mass% or less, the purity of the target recombinant spider silk protein (ADF3Kai-NN) was high in the proteins contained in the obtained supernatant, as compared with the case of using only purewater as the diluting solvent. Especially when the concentration of the ethanol in the dilution was 10 to 30 mass%, the purity of the target recombinant spider silk protein (ADF3Kai-NN) became still higher in the proteins contained in the obtained supernatant. Meanwhile, when the concentration of the ethanol in the dilution was 45 mass% or more, the purity of the target recombinant spider silk protein (ADF3Kai-NN) became low as compared with the case of using only purewater as the diluting solvent. The reason for this is considered to be that, when the concentration of the ethanol in the dilution is too high, the target recombinant spider silk protein (ADF3Kai-NN) aggregates.

FIG. 4 shows the results of SDS-PAGE of the proteins obtained in Examples 9-13. In FIG. 4, a lane M indicates a molecular-weight marker, a lane a indicates the protein contained in the lysate of the Escherichia coli of Example 9, and lanes b, c, d, e and f indicate the proteins contained in the supernatants obtained in Examples 9, 10, 11, 12 and 13, respectively. In FIG. 4, an arrow indicates the band of the protein corresponding to ADF3Kai-NN (molecular weight: about 47.5 kDa). It was confirmed from FIG. 4 and Table 4 that the supernatants containing the target recombinant spider silk protein (ADF3Kai-NN) were obtained by dissolving the Escherichia coli with the DMSO containing 0.5 M LiCl and diluting the lysate of the Escherichia coli with various kind of aqueous solvents. It also was found that, even when the 50 mM Tris buffer solutions at pH 3 to 9 were used as the diluting solvent, the target recombinant spider silk protein (ADF3Kai-NN) could be extracted as in the case of using pure water.

Further, it was found from the results of Examples 1, 2, 14 and 15 in Table 4 that the use of the DMSO containing an inorganic salt (LiCl) as the dissolving solvent made it possible to extract the recombinant spider silk protein (ADF3Kai-NN) with higher purity, as compared with the case of using only the DMSO not containing an inorganic salt.

### (Example 16)

The supernatant containing the protein ADF4Kai-W was collected in the same manner as in Example 2 except that about 0.1 g of dried bacterial cells of the Escherichia coli expressing the protein ADF4Kai-W was used.

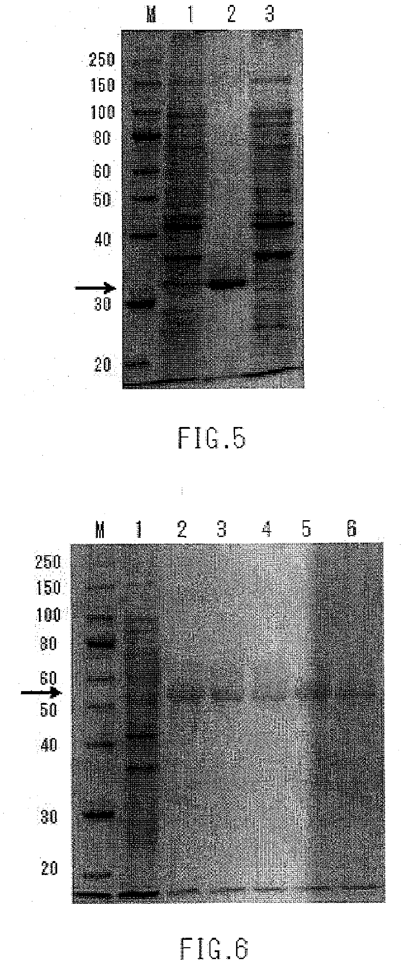

SDS-PAGE was performed as described above using the supernatant containing the protein ADF4Kai-W obtained in Example 16. After electrophoresis, the gel was stained with Coomassie Brilliant Blue (CBB). FIG. 5 shows the results. FIG. 5 also shows the results of SDS-PAGE using the lysate of the Escherichia coli in Example 16 and the precipitate obtained after collection of the supernatant containing the protein ADF4Kai-W As to the precipitate obtained after collection of the supernatant containing the protein ADF4Kai-W, SDS-PAGE was performed using a solution obtained by adding DMSO containing 2 M LiCl to the precipitate, followed by standing at 60°C for 30 minutes for dissolution.

In FIG. 5, a lane M indicates a molecular-weight marker, a lane 1 indicates the protein contained in the lysate of the Escherichia coli, a lane 2 indicates the protein contained in the supernatant, and a lane 3 indicates the protein contained in the precipitate. In FIG. 5, an arrow indicates the band of the protein corresponding to ADF4Kai-W (molecular weight: about 26.4 kDa). It was confirmed from FIG. 5 that the supernatant containing the target recombinant spider silk protein (ADF4Kai-W) was obtained by dissolving the Escherichia coli with the DMSO containing 0.5 M LiCl and diluting the lysate of the Escherichia coli with pure water.

### (Example 17)

(1) To about 0.1 g of dried bacterial cells of the Escherichia coli expressing the protein ADF3Kai-NN, 1 mL of DMSO containing 0.5 M LiCl was added so as to disperse the bacterial cells, followed by standing at 60°C for 30 minutes. Thus, a lysate of the Escherichia coli was obtained.
(2) The lysate of the Escherichia coli obtained was diluted with an equivalent amount of ultrapure water, and the obtained dilution was centrifuged at 20°C, 11,000 x g for 5 minutes using a centrifuge ("MX-305" manufactured by TOMY SEIKO Co., Ltd.). Then, the supernatant containing the protein ADF3Kai-NN was collected.

### (Examples 18-21)

Each of the supernatants containing the protein ADF3Kai-NN was collected in the same manner as in Example 17 except that an equivalent amount of a phosphate buffer solution shown in Table 5 below was used to dilute the lysate of the Escherichia coli.

SDS-PAGE was performed as described above using the supernatants containing the protein ADF3Kai-NN obtained in Examples 17-21. After electrophoresis, the gels were stained with Coomassie Brilliant Blue (CBB). FIG. 6 shows the results. The purity of the target protein (ADF3Kai-NN) was calculated by analyzing images of electrophoresis photographs of the protein after staining using analysis software ImageLab (Bio-RAD Laboratories, Inc.). Table 5 below shows the results. FIG. 6 also shows the results of SDS-PAGE using the lysate of the Escherichia coli in Example 17.

**[Table 5]**

| | Diluting solvent | Purity (%) |
|---|---|---|
| Example 17 | Ultrapure water | 63.6 |
| Example 18 | 50 mM sodium phosphate buffer solution (pH 3) | 56.8 |
| Example 19 | 50 mM sodium phosphate buffer solution (pH 5) | 53.1 |
| Example 20 | 50 mM sodium phosphate buffer solution (pH 7) | 55.5 |
| Example 21 | 50 mM sodium phosphate buffer solution (pH 9) | 54.7 |

FIG. 6 shows the results of SDS-PAGE of the proteins obtained in Examples 17-21. In FIG. 6, a lane M indicates a molecular-weight marker, a lane 1 indicates the protein contained in the lysate of the Escherichia coli of Example 17, and lanes 2, 3, 4, 5 and 6 indicate the proteins contained in the supernatants obtained in Examples 17, 18, 19, 20 and 21, respectively. In FIG. 6, an arrow indicates the band of the protein corresponding to ADF3Kai-NN (molecular weight: about 47.5 kDa). It was found from FIG. 6 and Table 5 that, even when the 50 mM sodium phosphate buffer solutions at pH 3 to 9 were used as the diluting solvent, the target recombinant spider silk protein (ADF3Kai-NN) could be extracted as in the case of using pure water.

### (Example 22)

The supernatant containing the protein Flag92-short2-UU was collected in the same manner as in Example 2 except that about 0.1 g of dried bacterial cells of the Escherichia coli expressing the protein Flag92-short2-UU was used.

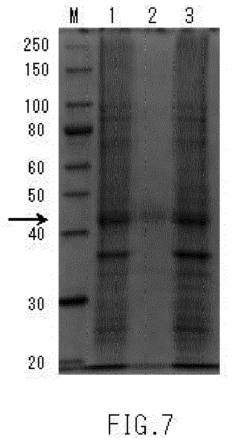

SDS-PAGE was performed as described above using the supernatant containing the protein Flag92-short2-UU obtained in Example 22. After electrophoresis, the gel was stained with Coomassie Brilliant Blue (CBB). FIG. 7 shows the results. FIG. 7 also shows the results of SDS-PAGE using the lysate of the Escherichia coli in Example 22 and the precipitate obtained after collection of the supernatant containing the protein Flag92-short2-UU. As to the precipitate obtained after collection of the supernatant containing the protein Flag92-short2-UU, SDS-PAGE was performed using a solution obtained by adding DMSO containing 2 M LiCl to the precipitate, followed by standing at 60°C for 30 minutes for dissolution.

In FIG. 7, a lane M indicates a molecular-weight marker, a lane 1 indicates the protein contained in the lysate of the Escherichia coli, a lane 2 indicates the protein contained in the supernatant, and a lane 3 indicates the protein contained in the precipitate. In FIG. 7, an arrow indicates the band of the protein corresponding to Flag92-short2-UU (molecular weight: about 36.9 kDa). It was confirmed from FIG. 7 that the supernatant containing the target recombinant spider silk protein (Flag92-short2-UU) was obtained by dissolving the Escherichia coli with the DMSO containing 0.5 M LiCl and diluting the lysate of the Escherichia coli with pure water.

### (Example 23)

(1) To about 0.1 g of dried bacterial cells of the Escherichia coli expressing the protein ADF3Kai-small-M, 1 ml of DMSO containing 1 M LiCl was added so as to disperse the bacterial cells, followed by dissolution at 60°C for 40 minutes. Thus, a lysate of the Escherichia coli was obtained.
(2) The lysate of the Escherichia coli obtained was diluted with an equivalent amount of ultrapure water, and the obtained dilution was centrifuged at 20°C, 11,000 x g for 5 minutes using a centrifuge ("MX-305" manufactured by TOMY SEIKO Co., Ltd.). Then, the supernatant containing the protein ADF3Kai-small-M was collected.

### (Example 24)

The supernatant containing the protein ADF3Kai-small-M was collected in the same manner as in Example 23 except that DMF containing 1 M LiCl was used instead of the DMSO containing 1 M LiCl.

### (Example 25)

The supernatant containing the protein ADF3Kai-small-M was collected in the same manner as in Example 23 except that DMA containing 1 M LiCl was used instead of the DMSO containing 1 M LiCl.

### (Example 26)

The supernatant containing the protein ADF3Kai-small-M was collected in the same manner as in Example 23 except that NMP containing 1 M LiCl was used instead of the DMSO containing 1 M LiCl.

SDS-PAGE was performed as described above using the supernatants containing the protein ADF3Kai-small-M obtained in Examples 23-26. After electrophoresis, the gels were stained with Oriole Fluorescent Gel Stain. FIG. 8 shows the results. The purity of the protein ADF3Kai-small-M in each of the supernatants was calculated by analyzing images of electrophoresis photographs of the protein after staining using analysis software ImageLab (Bio-RAD Laboratories, Inc.). Table 6 below shows the results.

The lower part of FIG. 8 shows the results of SDS-PAGE using the supernatants containing the protein ADF3Kai-small-M, and the upper part of FIG. 8 shows the results of SDS-PAGE using the corresponding lysates of the Escherichia coli. In FIG. 8, a lane M indicates a molecular-weight marker, and lanes 1, 2, 3 and 4 correspond to Examples 23, 24, 25 and 26, respectively. In FIG. 8, an arrow indicates the band of the protein corresponding to ADF3Kai-small-M (molecular weight: about 12.5 kDa).

**[Table 6]**

| | Dissolving solvent | Purity (%) |
|---|---|---|
| Example 23 | DMSO (containing 1 M LiCl) | 71.7 |
| Example 24 | DMF (containing 1 M LiCl) | 67.1 |
| Example 25 | DMA (containing 1 M LiCl) | 49.5 |
| Example 26 | NMP (containing 1 M LiCl) | 28 |

As can be seen from FIG. 8 and Table 6, when DMSO, DMF or DMA was used as the dissolving solvent, the purity of the target hydrophilic recombinant protein was high. Meanwhile, when NMP was used as the dissolving solvent, the purity of the target hydrophilic protein (recombinant protein) was low. By using the aprotic polar solvent not having a cyclic structure as the dissolving solvent, the recombinant protein (having HI of 0 or less) with higher purity could be extracted from a host cell expressing a hydrophilic recombinant protein having HI of 0 or less.

### (Example 27)

(1) To about 0.1 g of dried bacterial cells of the Escherichia coli expressing the protein Resilin-Kai, 1 ml of DMSO was added so as to disperse the bacterial cells, followed by dissolution at 60°C for 40 minutes. Thus, a lysate of the Escherichia coli (cell) was obtained.
(2) The lysate of the Escherichia coli obtained was diluted with an equivalent amount of ultrapure water, and the obtained dilution was centrifuged at 20°C, 11,000 x g for 5 minutes using a centrifuge ("MX-305" manufactured by TOMY SEIKO Co., Ltd.). Then, the supernatant containing the protein Resilin-Kai was collected.

### (Example 28)

The supernatant containing the protein Resilin-Kai was collected in the same manner as in Example 27 except that DMSO containing 1 M LiCl was used instead of the DMSO.

### (Example 29)

The supernatant containing the protein Resilin-Kai was collected in the same manner as in Example 27 except that DMF containing 1 M LiCl was used instead of the DMSO.

### (Example 30)

The supernatant containing the protein Resilin-Kai was collected in the same manner as in Example 27 except that NMP containing 1 M LiCl was used instead of the DMSO.

### (Example 31)

The supernatant containing the protein Resilin-Kai was collected in the same manner as in Example 27 except that DMI containing 1 M LiCl was used instead of the DMSO.

SDS-PAGE was performed as described above using the supernatants containing the protein Resilin-Kai obtained in Examples 27-31. After electrophoresis, the gels were stained with Oriole Fluorescent Gel Stain. The purity of Resilin-Kai in each of the supernatants was calculated by analyzing images of electrophoresis photographs of the protein after staining using analysis software ImageLab (Bio-RAD Laboratories, Inc.). Table 7 below shows the results.

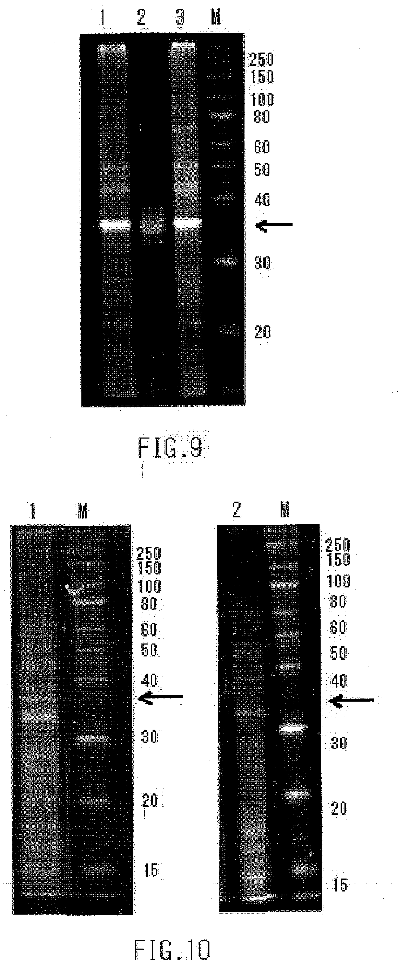

Further, FIG. 9 shows the results of SDS-PAGE performed using the supernatant containing the protein Resilin-Kai obtained in Example 28. FIG. 9 also shows the results of SDS-PAGE using the lysate of the Escherichia coli in Example 28 and the precipitate obtained after collection of the supernatant containing the protein Resilin-Kai. As to the precipitate obtained after collection of the supernatant containing the protein Resilin-Kai, SDS-PAGE was performed using a solution obtained by adding DMSO containing 2 M LiCl to the precipitate, followed by dissolution at 60°C for 30 minutes.

In FIG. 9, a lane M indicates a molecular-weight marker, a lane 1 indicates the protein contained in the lysate of the Escherichia coli, a lane 2 indicates the protein contained in the supernatant, and a lane 3 indicates the protein contained in the precipitate. In FIG. 9, an arrow indicates the band of the protein corresponding to Resilin-Kai (molecular weight: about 28.5 kDa). It was confirmed from FIG. 9 that the supernatant containing the target recombinant resilin (Resilin-Kai) was obtained by dissolving the Escherichia coli with the DMSO containing 1 M LiCl and diluting the lysate of the Escherichia coli with pure water.

**[Table 7]**

| | Dissolving solvent | Purity (%) |
|---|---|---|
| Example 27 | DMSO | 62.9 |
| Example 28 | DMSO (containing 1 M LiCl) | 69.7 |
| Example 29 | DMF (containing 1 M LiCl) | 60.6 |
| Example 30 | NMP (containing 1 M LiCl) | 25.4 |
| Example 31 | DMI (containing 1 M LiCl) | 22.0 |

As can be seen from the results of Table 7 above, when DMSO or DMF was used as the dissolving solvent, the purity of the target hydrophilic recombinant protein was high. Especially when the DMSO to which an inorganic salt was added was used as the dissolving solvent, the purity was still higher. Meanwhile, when NMP or DMI was used as the dissolving solvent, the purity of the target hydrophilic recombinant protein was low. It was confirmed that, by using the aprotic polar solvent not having a cyclic structure as the dissolving solvent, the recombinant protein (having HI of 0 or less) with still higher purity could be extracted from a host cell expressing a hydrophilic recombinant protein having HI of 0 or less.

### (Comparative Example 1)

(1) To about 0.1 g of dried bacterial cells of the Escherichia coli expressing the protein Resilin-Kai, 1 ml of isopropanol containing 1 M LiCl was added so as to disperse the bacterial cells, followed by dissolution at 60°C for 40 minutes. Thus, a lysate of the Escherichia coli (cell) was obtained.
(2) The lysate of the Escherichia coli obtained was diluted with an equivalent amount of ultrapure water, and the obtained dilution was centrifuged at 20°C, 11,000 x g for 5 minutes using a centrifuge ("MX-305" manufactured by TOMY SEIKO Co., Ltd.). Then, the supernatant was collected.

SDS-PAGE was performed as described above using the lysate of the Escherichia coli and the supernatant obtained in Comparative Example 1. After electrophoresis, the gels were stained with Oriole Fluorescent Gel Stain. FIG. 10 shows the results. In FIG. 10, a lane M indicates a molecular-weight marker, a lane 1 indicates the protein contained in the lysate of the Escherichia coli, and a lane 2 indicates the protein contained in the supernatant. In FIG. 10, an arrow indicates the position of the band of the protein corresponding to Resilin-Kai (molecular weight: about 28.5 kDa).

Further, the purity of the protein Resilin-Kai in the lysate of the Escherichia coli obtained in Comparative Example 1 and the purity of the supernatant were calculated by analyzing images of electrophoresis photographs of the protein after staining using analysis software ImageLab (Bio-RAD Laboratories, Inc.). As a result, the purity of the protein Resilin-Kai in the lysate of the Escherichia coli was 5.5%, and the purity of the protein Resilin-Kai in the supernatant was 0%.

In the case of using isopropanol, the protein Resilin-Kai could not be extracted from the Escherichia coli. That is, in the case of using a protic polar solvent, the recombinant protein (having HI of 0 or less) could not be extracted from a host cell expressing a hydrophilic recombinant protein having HI of 0 or less.

### (Example 32)

(1) To about 0.1 g of dried bacterial cells of the Escherichia coli expressing the protein Drosophila-Resilin-Kai, 1 ml of DMSO containing 1 M LiCl was added so as to disperse the bacterial cells, followed by standing at 60°C for 30 minutes for dissolution. Thus, a lysate of the Escherichia coli (cell) was obtained.
(2) The lysate of the Escherichia coli obtained was centrifuged at 20°C, 11,000 x g for 5 minutes using a centrifuge ("MX-305" manufactured by TOMY SEIKO Co., Ltd.). The supernatant of the lysate of the Escherichia coli obtained was diluted with an equivalent amount of ultrapure water, and the obtained dilution was centrifuged at 20°C, 11,000 x g for 5 minutes using a centrifuge ("MX-305" manufactured by TOMY SEIKO Co., Ltd.). Then, the supernatant containing the protein Drosophila-Resilin-Kai was collected.

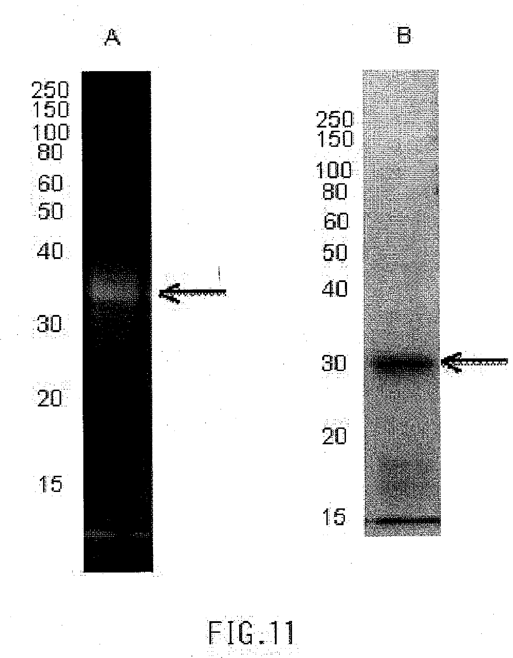

SDS-PAGE was performed as described above using the supernatant containing the protein Drosophila-Resilin-Kai obtained in Example 32. After electrophoresis, the gel was stained with Oriole Fluorescent Gel Stain. FIG. 11A shows an electrophoresis photograph of the protein after staining. In FIG. 11A, an arrow indicates the band of the protein corresponding to Drosophila-Resilin-Kai (molecular weight: about 28.5 kDa). It was confirmed from FIG. 11A that the supernatant containing the target recombinant resilin protein (Drosophila-Resilin-Kai) was obtained by dissolving the Escherichia coli with the DMSO containing 1.0 M LiCl and diluting the lysate of the Escherichia coli with pure water. As a result of analyzing images of electrophoresis photographs of the protein after staining using analysis software ImageLab (Bio-RAD Laboratories, Inc.), the purity of Drosophila-Resilin-Kai in the supernatant was 69.7%.

### (Example 33)

(1) To about 0.1 g of dried bacterial cells of the Escherichia coli expressing the protein Collagen-type4-Kai, 1 ml of DMSO containing 1 M LiCl was added so as to disperse the bacterial cells, followed by standing at 60°C for 30 minutes for dissolution. Thus, a lysate of the Escherichia coli (cell) was obtained.
(2) The lysate of the Escherichia coli obtained was centrifuged at 20°C, 11,000 x g for 5 minutes using a centrifuge ("MX-305" manufactured by TOMY SEIKO Co., Ltd.). The supernatant of the lysate of the Escherichia coli obtained was diluted with an equivalent amount of ultrapure water, and the obtained dilution was centrifuged at 20°C, 11,000 x g for 5 minutes using a centrifuge ("MX-305" manufactured by TOMY SEIKO Co., Ltd.). Then, the supernatant containing the protein Collagen-type4-Kai was collected.

SDS-PAGE was performed as described above using the supernatant containing the protein Collagen-type4-Kai obtained in Example 33. After electrophoresis, the gel was stained with Coomassie Brilliant Blue (CBB). FIG. 11B shows the results. In FIG. 11B, an arrow indicates the band of the protein corresponding to Collagen-type4-Kai (molecular weight: about 24.5 kDa). It was confirmed from FIG. 11B that the supernatant containing the target recombinant collagen protein (Collagen-type4-Kai) was obtained by dissolving the Escherichia coli with the DMSO containing 1.0 M LiCl and diluting the lysate of the Escherichia coli with pure water. As a result of analyzing images of electrophoresis photographs of the protein after staining using analysis software ImageLab (Bio-RAD Laboratories, Inc.), the purity of Collagen-type4-Kai in the supernatant was 43.7%.

### Industrial Applicability

According to the present invention, it is possible to extract hydrophilic proteins from hosts expressing hydrophilic proteins having a hydropathy index of 0 or less as recombinant proteins.

### Sequence Listing Free Text

SEQ ID NOS: 1, 2, 3, 6, 8, 10, 12, 13, 15, 16 amino acid sequences
SEQ ID NOS: 4, 5, 7, 9, 11, 14, 17 base sequences

## Claims

1. A method for extracting a hydrophilic recombinant protein from a host expressing a hydrophilic recombinant protein,
the method comprising:
a lysate acquisition step in which a dissolving solvent is added to the host expressing a hydrophilic recombinant protein so as to obtain a host cell lysate; and
a step in which a diluting solvent is added to the host cell lysate obtained in the lysate acquisition step, an insoluble substance is separated from the obtained dilution, and a supernatant containing the hydrophilic recombinant protein is collected,
wherein the hydrophilic recombinant protein has a hydropathy index of 0 or less,
the dissolving solvent is an aprotic polar solvent having a dipole moment of 3.0 D or more, and
the diluting solvent is an aqueous solvent.

2. The method for extracting a hydrophilic recombinant protein according to claim 1, wherein the dissolving solvent is an aprotic polar solvent not having a cyclic structure.

3. The method for extracting a hydrophilic recombinant protein according to claim 1 or 2, wherein the dissolving solvent is one or more kinds of aprotic polar solvent selected from the group consisting of dimethyl sulfoxide, N,N-dimethylformamide, and N,N-dimethylacetamide.

4. The method for extracting a hydrophilic recombinant protein according to any one of claims 1 to 3, wherein the dissolving solvent is a solvent obtained by adding an inorganic salt to the aprotic polar solvent.

5. The method for extracting a hydrophilic recombinant protein according to any one of claims 1 to 4, wherein the diluting solvent is one kind of aqueous solvent selected from the group consisting of (1) water, (2) a mixture of water and ethanol, and (3) a water-soluble buffer solution.

6. The method for extracting a hydrophilic recombinant protein according to any one of claims 1 to 5, wherein the amount of the dissolving solvent which is added is 0.5 times or more the mass of the host by volume (mL)/mass (g) ratio.

7. The method for extracting a hydrophilic recombinant protein according to any one of claims 1 to 6, wherein the amount of the diluting solvent which is added is 0.5 times or more an amount of the host cell lysate by volume ratio.

8. The method for extracting a hydrophilic recombinant protein according to any one of claims 1 to 7, wherein the hydrophilic protein is a protein selected from the group consisting of a recombinant spider silk protein, a recombinant resilin, and a recombinant collagen.

## Patentansprüche

1. Verfahren zur Extraktion eines hydrophilen rekombinanten Proteins aus einem Wirt, der ein hydrophiles rekombinantes Protein exprimiert,
wobei das Verfahren umfasst:
einen Lysat-Akquisitionsschritt, bei welchem dem Wirt ein auflösendes Lösungsmittel zugesetzt wird, das ein hydrophiles rekombinantes Protein exprimiert, um ein Wirtszelllysat zu erhalten; und
einen Schritt, bei dem ein verdünnendes Lösungsmittel dem in dem Lysat-Akquisitionsschritt erhaltenen Wirtszelllysat zugesetzt wird, eine unlösliche Substanz von der erhaltenen Verdünnung abgetrennt wird, und ein das hydrophile rekombinante Protein enthaltender Überstand gesammelt wird,
wobei das hydrophile rekombinante Protein einen Hydropathieindex von 0 oder weniger aufweist,
das auflösende Lösungsmittel ein aprotisches polares Lösungsmittel ist, das ein Dipolmoment von 3,0 D oder mehr aufweist, und
das verdünnende Lösungsmittel ein wässriges Lösungsmittel ist.

2. Verfahren zur Extraktion eines hydrophilen rekombinanten Proteins nach Anspruch 1, wobei das auflösende Lösungsmittel ein aprotisches polares Lösungsmittel ist, das keine zyklische Struktur aufweist.

3. Verfahren zur Extraktion eines hydrophilen rekombinanten Proteins nach einem der Ansprüche 1 oder 2, wobei das auflösende Lösungsmittel eine oder mehrere Arten aprotischen polaren Lösungsmittels ist, ausgewählt aus der Gruppe, bestehend aus Dimethylsulfoxid, N,N-Dimethylformamid und N,N-Dimethylacetamid.

4. Verfahren zur Extraktion eines hydrophilen rekombinanten Proteins nach einem der Ansprüche 1 bis 3, wobei das auflösende Lösungsmittel ein Lösungsmittel ist, das durch Zusatz eines anorganischen Salzes zu dem aprotischen polaren Lösungsmittel erhalten wird.

5. Verfahren zur Extraktion eines hydrophilen rekombinanten Proteins nach einem der Ansprüche 1 bis 4, wobei das verdünnende Lösungsmittel eine Art wässriges Lösungsmittel ist, ausgewählt aus der Gruppe, bestehend aus (1) Wasser, (2) einem Gemisch aus Wasser und Ethanol und (3) einer wasserlöslichen Pufferlösung besteht.

6. Verfahren zur Extraktion eines hydrophilen rekombinanten Proteins nach einem der Ansprüche 1 bis 5, wobei die Menge des auflösenden Lösungsmittels, das zugesetzt wird, das 0,5-fache oder mehr der Masse des Wirts, ausgedrückt als Verhältnis Volumen (mL)/Masse (g), beträgt.

7. Verfahren zur Extraktion eines hydrophilen rekombinanten Proteins nach einem der Ansprüche 1 bis 6, wobei die Menge verdünnenden Lösungsmittels, das zugesetzt wird, das 0,5-fache oder mehr einer Menge des Wirtszelllysats im Volumenverhältnis beträgt.

8. Verfahren zur Extraktion eines hydrophilen rekombinanten Proteins nach einem der Ansprüche 1 bis 7, wobei das hydrophile Protein ein Protein ist, ausgewählt aus der Gruppe, bestehend aus einem rekombinanten Spinnenseidenprotein, einem rekombinanten Resilin und einem rekombinanten Kollagen.

## Revendications

1. Procédé d'extraction d'une protéine recombinante hydrophile à partir d'un hôte exprimant une protéine recombinante hydrophile,
le procédé comprenant :
une étape d'obtention de lysat dans laquelle un solvant de dissolution est ajouté à l'hôte exprimant une protéine recombinante hydrophile afin d'obtenir un lysat de cellule hôte ; et
une étape dans laquelle un solvant de dilution est ajouté au lysat de cellule hôte obtenu dans l'étape d'obtention de lysat, une substance insoluble est séparée de la dilution obtenue, et un surnageant contenant la protéine recombinante hydrophile est collecté,
dans lequel la protéine recombinante hydrophile présente un indice d'hydropathie de 0 ou moins,
le solvant de dissolution est un solvant polaire aprotique présentant un moment dipolaire de 3,0 D ou plus, et
le solvant de dilution est un solvant aqueux.

2. Procédé d'extraction d'une protéine recombinante hydrophile selon la revendication 1, dans lequel le solvant de dissolution est un solvant polaire aprotique n'ayant pas une structure cyclique.

3. Procédé d'extraction d'une protéine recombinante hydrophile selon la revendication 1 ou 2, dans lequel le solvant de dissolution est un ou plusieurs types de solvant polaire aprotique sélectionnés à partir du groupe constitué par du diméthylsulfoxyde, du N,N-diméthylformamide, et du N,N-diméthylacétamide.

4. Procédé d'extraction d'une protéine recombinante hydrophile selon l'une quelconque des revendications 1 à 3, dans lequel le solvant de dissolution est un solvant obtenu en ajoutant un sel inorganique au solvant polaire aprotique.

5. Procédé d'extraction d'une protéine recombinante hydrophile selon l'une quelconque des revendications 1 à 4, dans lequel le solvant de dilution est un type de solvant aqueux sélectionné dans le groupe constitué par (1) de l'eau, (2) un mélange d'eau et d'éthanol, et (3) une solution tampon hydrosoluble.

6. Procédé d'extraction d'une protéine recombinante hydrophile selon l'une quelconque des revendications 1 à 5, dans lequel la quantité du solvant de dissolution qui est ajoutée représente 0,5 fois ou plus la masse de l'hôte selon un rapport volume (ml)/masse (g).

7. Procédé d'extraction d'une protéine recombinante hydrophile selon l'une quelconque des revendications 1 à 6, dans lequel la quantité du solvant de dilution qui est ajoutée représente 0,5 fois ou plus une quantité du lysat de cellule hôte selon un rapport en volume.

8. Procédé d'extraction d'une protéine recombinante hydrophile selon l'une quelconque des revendications 1 à 7, dans lequel la protéine hydrophile est une protéine sélectionnée dans le groupe constitué par une protéine recombinante de soie d'araignée, une résiline recombinante, et un collagène recombinant.
